(19) Europäisches Patentamt
European Patent Office
Office européen des brevets

(11) **EP 2 404 543 A1**

(12) **EUROPEAN PATENT APPLICATION**

(43) Date of publication:
**11.01.2012 Bulletin 2012/02**

(51) Int Cl.:
*A61B 1/06* (2006.01)   *A61B 5/00* (2006.01)
*A61B 1/04* (2006.01)   *A61B 1/07* (2006.01)

(21) Application number: **11179380.8**

(22) Date of filing: **22.05.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: 22.05.2008 JP 2008134069
22.05.2008 JP 2008134070
22.05.2008 JP 2008134071
22.05.2008 JP 2008134072
22.05.2008 JP 2008134073

(62) Document number(s) of the earlier application(s) in accordance with Art. 76 EPC:
**09006889.1 / 2 123 213**

(71) Applicant: **Fujifilm Corporation**
**Minato-ku**
**Tokyo (JP)**

(72) Inventor: **Ishii, Shuichi**
**Saitama (JP)**

(74) Representative: **Klunker, Hans-Friedrich**
**Klunker Schmitt-Nilson Hirsch**
**Patentanwälte**
**Destouchesstrasse 68**
**80796 München (DE)**

Remarks:
This application was filed on 30-08-2011 as a divisional application to the application mentioned under INID code 62.

(54) **Fluorescent image obtainment method and apparatus, fluorescence endoscope, and excitation-light unit**

(57)     A fluorescent image obtainment apparatus includes a light illumination means (130, 150) that illuminates a region to be observed with illumination-light and excitation-light at the same time, and an imaging means (110) that captures an image composed of light reflected from the region illuminated with the illumination-light and an image composed of fluorescence output from the region illuminated with the excitation-light. Further, the apparatus includes an image processing means (170) that produces an ordinary image based on the image of the reflection light and a fluorescent image based on the image of fluorescence, and a light amount control means (186) that controls the light amount of the illumination-light so that a representative luminance value of the ordinary image becomes a predetermined luminance value and the light amount of the excitation-light so that the ratio of the light amount of the excitation-light to that of the illumination-light becomes a predetermined ratio.

**FIG.1**

EP 2 404 543 A1

## Description

BACKGROUND OF THE INVENTION

Field of the Invention

The present invention relates to a fluorescent image obtainment method, a fluorescent image obtainment apparatus, a fluorescence endoscope apparatus, and an excitation light unit. Particularly, the present invention relates to a fluorescent image obtainment method, a fluorescent image obtainment apparatus and a fluorescence endoscope apparatus that produce a fluorescent image by obtaining an image including fluorescence output from a region to be observed by illumination with excitation light. Further, the present invention relates to an excitation light unit that outputs excitation light to produce the fluorescent image.

Description of the Related Art

Conventionally, endoscope apparatuses for observing tissue in the body cavities of patients (in vivo) have been widely known. Further, electronic endoscopes that obtain ordinary images by imaging regions to be observed in the body cavities while the regions are illuminated with white light and that display the obtained ordinary images on monitors (displays) are widely used.

In addition to observation of the regions to be observed by illuminating the regions with white light, fluorescent image obtainment apparatuses that are used as fluorescence endoscope apparatuses are well known. The fluorescence endoscope apparatuses capture fluorescent images by receiving autofluorescence output from the regions to be observed by illuminating the regions with excitation light. Further, the fluorescence endoscope apparatuses display the fluorescent images on monitors together with the ordinary images. The autofluorescence is output from endogenous fluorescent substance in living tissue (tissue in vivo). For example, when the region to be observed is the mucous membrane of airway, most of the autofluorescence is thought to be output from the submucosa. Examples of the endogenous fluorescent substance are riboflavin, tryptophan, tyrosine, NADH, NADPH, porphyrin, collagen, elastin, fibronectin, FAD and the like.

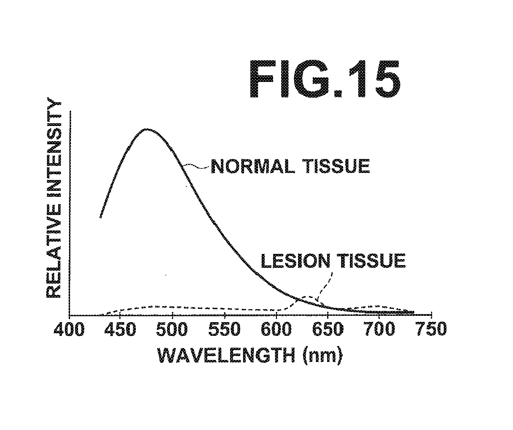

Further, it is known that when a region to be observed, such as living tissue, is illuminated with excitation light of a predetermined wavelength band, the light intensity and the spectral shape of autofluorescence output from an autofluorescent substance that is present in the region to be observed differ depending on whether the autofluorescence is output from normal tissue or lesion tissue, as illustrated in Figure 15. A fluorescence endoscope apparatus that utilizes this phenomenon is known (for example, please refer to U.S. Patent No. 6,465,968). In this fluorescence endoscope apparatus, a region to be observed is illuminated with excitation light of a predetermined wavelength, and autofluorescence output from the region to be observed is detected to produce a fluorescent image. The reason why the autofluorescence output from the lesion tissue is weaker than the autofluorescence output from the normal tissue is presumed to be thickening of the mucous epithelium of the lesion tissue, consumption of the endogenous fluorescent substance in the lesion tissue, an increase in fluorescence absorption substance in the lesion tissue and the like.

Further, a fluorescent image obtainment apparatus that produces a fluorescent image, for example, by administering an oncotropic photosensitive substance (ATX-S10, 5-ALA, NPe6, HAT-D01, Photofrin-2 or the like), as a fluorescent dye, to a patient (a subject to be examined) in advance is well known. The oncotropic photosensitive substance outputs fluorescence by being excited by light. The administered fluorescent dye is absorbed by a tumor region, such as a cancer. The tumor region that has absorbed the fluorescent dye is illuminated with excitation light in an excitation wavelength range of the fluorescent dye, and fluorescence output from the fluorescent dye that has accumulated in the tumor region is detected to produce the fluorescent image.

In these fluorescent image obtainment apparatuses, various comparative analysis methods have been proposed so that users (observers, or examiners, or doctors) can obtain more accurate information about the tissue characteristics based on the fluorescence information. For example, when the intensity of autofluorescence output from a region to be observed, such as living tissue, by illumination with excitation light is obtained as a fluorescence image, and fluorescence information based on the fluorescence image is displayed, if the region to be observed is a normal region, the intensity of fluorescence output from the region to be observed is substantially proportional to the illumination intensity of the excitation light. However, the illumination intensity of the excitation light decreases in inverse proportion to the square of a distance. Therefore, there are cases in which fluorescence received from a lesion tissue that is located close to a light source is higher than fluorescence received from a normal tissue that is located far from the light source. Therefore, it is impossible to accurately judge the tissue characteristics of the region to be observed only by the information about the intensity of fluorescence.

To solve such problems, U.S. Patent Application Publication No. 20030216626 proposes a fluorescent image obtainment apparatus that diagnoses the tissue characteristic of the living body. In the fluorescent image obtainment apparatus, the region to be observed is illuminated with reference light of a wavelength band that is different from the wavelength band of the excitation light. Further, the intensity of light reflected from the region to be observed that has been illuminated with the reference light is detected. Further, diagnosis information representing a lesion of the living tissue is obtained based on fluorescence yield represented by the ratio of the intensity of fluorescence to the intensity of the reflection light of the reference light. Further, the region of the lesion, which is the diagnosis information, is displayed in a different color, such as red, in a displayed fluorescent image.

In these fluorescent image obtainment apparatuses, when the distance from the illumination light output end to the region to be observed is long, a sufficient amount of illumination light does not reach the region to be observed, and there is a risk that a dark image is obtained. Therefore, when a motion image (dynamic image or video image) is obtained, the lightness of the image, such as an average luminance value of the image, is constantly detected to control the light amount of the illumination light so that constant lightness can be obtained.

However, in an apparatus that obtains an ordinary image and a fluorescent image by illuminating the region to be observed with both of the illumination light and the excitation light at the same time, as described above, when the ordinary image is superimposed on the fluorescent image and displayed, if the light amount of only the illumination light is controlled, it is not sufficient. For example, when the light of the illumination light is increased, only the ordinary image becomes light, and the fluorescent image remains dark. Therefore, it becomes difficult to identify the fluorescent image in the superimposed image. Further, when the light amount of the illumination light is reduced, only the ordinary image becomes dark, and the fluorescent image remains light. Therefore, the lightness of the ordinary image and that of the fluorescent image become imbalanced, and it becomes difficult to observe the region to be observed in the image.

Meanwhile, when a fluorescent image is obtained, even if the distance from the excitation light output end to the region to be observed is short, a sufficient amount of fluorescence is not obtained in some cases, because the light amount of fluorescence output from the region to be observed differs according the tissue characteristic of the region to be observed. Therefore, if the light amount of the excitation light is controlled based on the lightness of the fluorescent image in a manner similar to the control of the illumination light, there is a problem that the reliability of the fluorescent image becomes lower.

Further, when the light amount of the excitation light is tried to be accurately controlled, the drive current of the white light source is relatively high compared with the drive current of the excitation light source. Therefore, noise caused by the drive current of the white light source becomes a problem.

Further, for example, U.S. Patent Application Publication No. 20040162492 proposes a fluorescent image obtainment apparatus that obtains an ordinary image by outputting white light and that obtains a fluorescent image by outputting excitation light, as described above.

In the fluorescent image obtainment apparatus disclosed in U.S. Patent Application Publication No. 20040162492, the white light and the excitation light are alternately output by hardware spectral structure, and an ordinary image and a fluorescent image are obtained synchronously with the output of the white light and the excitation right.

Further, in a fluorescent image obtainment apparatus proposed in Japanese Patent No. 3718024, white light and excitation light are combined on the same optical axis, and the combined light is guided to a living body that has absorbed light-sensitive substance. Further, the wavelength band of the excitation light is removed from light information output from the living body. Further, a wavelength division means transmits light in a wavelength band of fluorescence and reflects light in the other wavelength band. Further, a fluorescent image is obtained by capturing the divided light.

Further, Japanese Unexamined Patent Publication 2002-143079 proposes a method in which subtraction operation is performed between a fluorescent image signal and an ordinary image signal, and the difference is emphasized by amplifying the difference. Further, U. S. Patent Application Publication No. 20060256191 proposes a method in which a fluorescent image signal and an ordinary image signal are combined and displayed to improve the accuracy of screening.

However, in the apparatus that obtains both of the ordinary image signal and the fluorescent image signal by outputting the white light and the excitation light, respectively, as described above, if the white light and the excitation light are alternately output by hardware spectral structure as disclosed in U.S. Patent Application Publication No. 20040162492, the size of the apparatus becomes large, and the cost of the apparatus increases. Further, the case of providing the wavelength division means as disclosed in Japanese Patent No. 3718024 has similar problems.

Further, in recent years, in the field of electronic endoscope apparatuses using solid-state imaging devices, an electronic endoscope apparatus with a built-in narrow-band-pass filter (Narrow Band Imaging - NBI) has been drawing attention. The electronic endoscope apparatus with a built-in narrow-band-pass filter performs spectral imaging by combining narrow-band-pass filters based on the spectral reflectance of a digestive organ, such as the mucosa of stomach (gastric mucosa) . In this apparatus, a narrow (wavelength) band pass filter is provided instead of a plane-sequential R (red) - G (green) - B (blue) rotary filter. Further, illumination light is sequentially output through the narrow-band-pass filter, and processing similar to the processing of R, G, and B (RGB) signals is performed while the weighting applied to signals obtained with these kinds of illumination light is changed. Accordingly, a spectral image is obtained. When such a spectral image is used, it is possible to extract a minute structure in a digestive organ, such as the stomach (gaster) and the large intestine, which has not been obtained in the conventional method.

Meanwhile, Japanese Unexamined Patent Publication No. 2003-093336 and U.S. Patent Application Publication No. 20070183162 propose a technique of forming a spectral image by operation processing based on an image signal obtained by imaging a region to be observed by illuminating the region with white light in a simultaneous method instead of the aforementioned plane-sequential method that uses the narrow-band-pass filter. In the simultaneous method, a minute mosaic color filter is arranged on a solid-state imaging device. Japanese Unexamined Patent Publication No. 2003-093336 discloses a method for obtaining spectral data of the region to be observed that depends on none of the type of the illumination light, the unique spectral characteristic of the imaging system and the like. In the method, estimation matrix data that takes the spectral characteristic of the illumination light and the spectral characteristic of the whole imaging system including the color sensitivity characteristic of the imaging device, the transmissivity of the color filter or the like, into consideration is obtained. Further, the spectral data is obtained by performing operation using the RGB image signals obtained by imaging by the imaging device and the estimation matrix data.

Further, in the fluorescent image obtainment apparatus as described above, obtainment of the fluorescent image by illuminating the region to be observed only with the excitation light and obtainment of the ordinary image by illuminating the region to be observed only with the illumination light are performed in time-division in some cases. When the fluorescent image and the ordinary image are obtained by time division, the number of frames of the fluorescent images and the ordinary images per unit time period becomes small. Therefore, when a motion image is displayed, there is a problem that an efficient motion image is not displayed.

Further, in the fluorescence endoscope apparatus as described above, it is necessary to provide a light source for outputting excitation light in addition to a light source for outputting illumination light. Therefore, when a user (doctor) who uses an ordinary endoscope wishes to observe a fluorescent image, he/she needs to additionally purchase an expensive fluorescence endoscope apparatus. However, observation of the fluorescent image is performed only to supplement the observation of the ordinary image in many cases, and the frequency of observing the fluorescent image is much smaller than the frequency of observing the ordinary image. Therefore, many users (doctors) desire to use the electronic endoscope apparatuses that they already have to observe the fluorescent images without purchasing new fluorescent endoscope apparatuses.

SUMMARY OF THE INVENTION

In view of the foregoing circumstances, it is an object of the present invention to improve the balance between the lightness of an ordinary image and the lightness of a fluorescent image in a fluorescent image obtainment method and apparatus that obtains the ordinary image and the fluorescent image by illuminating the region to be observed with illumination light and excitation light. Further, it is another object of the present invention to improve the reliability of the obtained fluorescent image.

Further, it is another object of the present invention to provide a fluorescent image obtainment method and apparatus that can improve the noise resistance of the excitation light source.

Further, it is another object of the present invention to provide a fluorescent image obtainment method and apparatus that can simplify the structure of the whole apparatus and the cost of the apparatus.

Further, it is another object of the present invention to provide a fluorescent image obtainment method and apparatus that can produce an efficient display image even when a motion image is displayed.

Meanwhile, the inventor of the present invention has studied whether an existing electronic endoscope apparatus can function as a fluorescent image endoscope apparatus by attaching a detachable excitation light unit including an excitation light source to the existing electronic endoscope apparatus. As the excitation light source, a high-luminance LED, a semiconductor laser or the like is used. Since the user of the fluorescence endoscope apparatus may not be used to handling of the light source, such as the LED and the laser, it is essential that the safety of the user is ensured.

In these circumstances, it is another object of the present invention to provide a fluorescence endoscope apparatus and an excitation light unit that are user-friendly, and in which the safety of the users is assured.

A first fluorescent image obtainment method of the present invention is a fluorescent image obtainment method comprising the steps of:

capturing an image composed of light reflected from a region to be observed, the region having been illuminated with illumination light, and an image composed of fluorescence output from the region to be observed, the region having been illuminated with excitation light at the same time as illumination with the illumination light;
producing an ordinary image based on the captured image composed of the reflection light; and
producing a fluorescent image based on the captured image composed of fluorescence, wherein the light amount of the illumination light is controlled so that a representative luminance value of the ordinary image (a value representing the luminance of the ordinary image or the like) becomes a predetermined luminance value, and wherein the light amount of the excitation light is controlled so that the ratio of the light amount of the excitation light to the light amount of the illumination light becomes a predetermined ratio.

A second fluorescent image obtainment method of the present invention is a fluorescent image obtainment method comprising the steps of:

driving an illumination light source means so as to continuously output illumination light;

pulse-driving an excitation light source means, based on a pulse signal, so as to output pulsed excitation light;

capturing an image composed of light reflected from a region to be observed by illumination with the illumination light, and an image including fluorescence output from the region to be observed by illumination with excitation light;

producing an ordinary image based on the captured image composed of the reflection light; and

producing a fluorescent image based on the captured image including fluorescence, wherein the light amount of the illumination light is controlled so that a representative luminance value of the ordinary image becomes a predetermined luminance value, and wherein the light amount of the excitation light is controlled by controlling the pulse signal so that the ratio of the light amount of the excitation light to the light amount of the illumination light becomes a predetermined ratio.

A third fluorescent image obtainment method of the present invention is a fluorescent image obtainment method comprising the steps of:

driving an illumination light source means so as to continuously output illumination light;

pulse-driving an excitation light source means so as to output pulsed excitation light;

continuously illuminating a region to be observed with the illumination light;

illuminating a region that is substantially the same as the region illuminated with the illumination light with the pulsed excitation light;

capturing a first image composed of light reflected from the region to be observed in a time period when the region to be observed is illuminated only with the illumination light in the process of continuously illuminating the region to be observed with the illumination light and illuminating substantially the same region with the pulsed excitation light;

capturing a second image composed of light reflected from the region to be observed and fluorescence output from the region to be observed in a time period when the region to be observed is illuminated with both of the illumination light and the excitation light in the process of continuously illuminating the region to be observed with the illumination light and illuminating substantially the same region with the pulsed excitation light;

producing an ordinary image based on the first image; and

producing a fluorescent image based on the first image and the second image.

A fourth fluorescent image obtainment method of the present invention is a fluorescent image obtainment method comprising the steps of:

capturing an image composed of light reflected from a region to be observed, the region having been illuminated with illumination light, and fluorescence output from the region to be observed, the region having been illuminated with excitation light at the same time as illumination with the illumination light;

calculating estimated spectral data of a specific fluorescence wavelength band for each pixel of image signals of the captured image, the specific fluorescence wavelength band including at least an approximate center wavelength band of the fluorescence and the estimated spectral data being calculated based an image signal value of each of the pixels and an estimation matrix for calculating the estimated spectral data, and the estimation matrix having been stored in advance;

obtaining, based on the estimated spectral data of the specific fluorescence wavelength band, information that reflects the radiation intensity of the fluorescence output from the region to be observed; and

producing a fluorescent image based on the information that reflects the radiation intensity of the fluorescence.

A first fluorescent image obtainment apparatus of the present invention is a fluorescent image obtainment apparatus comprising:

a light illumination means that illuminates a region to be observed with illumination light and excitation light at the same time;

an imaging means that captures an image composed of light reflected from the region to be observed, the region having been illuminated with the illumination light, and an image composed of fluorescence output from the region to be observed, the region having been illuminated with the excitation light;

an image processing means that produces an ordinary image based on the image composed of the reflection light, the image having been captured by the imaging means, and a fluorescent image based on the image composed of fluorescence, the image having been captured by the imaging means; and

a light amount control means that controls the light amount of the illumination light so that a representative luminance value of the ordinary image becomes a predetermined luminance value, and that controls the light amount of the excitation light so that the ratio of the light amount of the excitation light to the light amount of the illumination light becomes a predetermined ratio.

In the first fluorescent image obtainment of the present invention, the imaging means may separately obtain an image composed of light reflected from a region to be observed that has been illuminated with illumination light and an image composed of fluorescence output from the region to be observed that has been illuminated with excitation light. Alternatively, the imaging means may obtain an image in which an image composed of reflection light and an image composed of fluorescence are superimposed one on the other.

Further, the first fluorescent image obtainment apparatus of the present invention may further include:

a storage unit that stores an estimation matrix for pseudo-spectral image processing; and
an estimated spectral data calculation means that uses an image signal output from the imaging means and the estimation matrix, and calculates, for each pixel of the image signal, estimated spectral data. Further, the image processing means may obtain, based on the estimated spectral data for each of the pixels, estimated spectral data of a specific fluorescence wavelength band that includes at least a center wavelength band of the fluorescence, and produce the fluorescent image based on the estimated spectral data of the specific fluorescence wavelength band.

Further, the estimation matrix may take the spectral characteristic of illumination light and the spectral characteristic of the imaging means into consideration. Further, the estimation matrix may be used to calculate the spectral data of the region to be observed by performing matrix operation using the image signal. Alternatively, the estimation matrix may take the spectral characteristic of both of the illumination light and the excitation light and the spectral characteristic of the imaging means into consideration. Further, the estimation matrix may be used to calculate the spectral data of the region to be observed and the fluorescent spectrum of fluorescence output from the region to be observed by performing matrix operation using the image signal.

Further, the image processing means may:

calculate, based on the estimated spectral data of the specific fluorescence wavelength band, pseudo-fluorescence intensity, which is the intensity of light in the specific fluorescence wavelength band;
obtain estimated spectral data of a reference wavelength band that does not include the specific fluorescence wavelength band;
calculate, based on the estimated spectral data of the reference wavelength band, reference light intensity, which is the intensity of light in the reference wavelength band;
calculate a pseudo-fluorescence yield by dividing the pseudo-fluorescence intensity by the reference light intensity; and

produce the fluorescent image based on the pseudo-fluorescence yield.

A second fluorescent image obtainment apparatus of the present invention is a fluorescent image obtainment apparatus comprising:

an illumination light source means that continuously outputs illumination light;
an excitation light source means that is pulse-driven based on a pulse signal to output pulsed excitation light;
an illumination means that illuminates substantially the same position of a region to be observed with the illumination light and the excitation light;
an imaging means that captures an image composed of light reflected from the region to be observed by illumination with the illumination light, and an image including fluorescence output from the region to be observed by illumination with the excitation light;
an image processing means that produces an ordinary image based on the image composed of the reflection light, the image having been captured by the imaging means, and that produces a fluorescent image based on the image including fluorescence, the image having been captured by the imaging means; and
a light amount control means that controls the light amount of the illumination light so that a representative luminance value of the ordinary image becomes a predetermined luminance value, and that controls the light amount of the excitation light by controlling the pulse signal so that the ratio of the light amount of the excitation light to the light amount of the illumination light becomes a predetermined ratio.

Further, in the second fluorescent image obtainment apparatus of the present invention, the excitation light source means may output, at a predetermined cycle, one or a plurality of beams of pulsed excitation light in a predetermined time period in which the excitation light can be output. Further, the light amount control means may control the light amount of the excitation light by changing the number of beams of the pulsed excitation light in the predetermined time period in which the excitation light can be output and/or the light output time period of the pulsed excitation light in the predetermined time period in which the excitation light can be output.

Further, each of the first and second fluorescent image obtainment apparatuses of the present invention may further include a setting means that sets the ratio of the light amount of the excitation light to the light amount of the illumination light by an input operation.

A third fluorescent image obtainment apparatus of the present invention is a fluorescent image obtainment apparatus comprising:

an illumination light source means that continuously outputs illumination light;

an excitation light source means that is pulse-driven based on a pulse signal to output pulsed excitation light;

an illumination means that illuminates substantially the same position of a region to be observed with the illumination light and the excitation light;

an imaging means that captures a first image composed of light reflected from the region to be observed in a time period when the region to be observed is illuminated only with the illumination light and that captures a second image composed of light reflected from the region to be observed and fluorescence output from the region to be observed in a time period when the region to be observed is illuminated with both of the illumination light and the excitation light; and

an image processing means that produces an ordinary image based on the first image and that produces, based on the ordinary image and a combined image that is based on the second image, a fluorescent image.

Further, in the third fluorescent image obtainment apparatus of the present invention, the image processing means may produce an image for display by performing an operation by using at least two of the ordinary image, the fluorescent image and the combined image.

Further, the image processing means may produce, based on the luminance ratio between the ordinary image and the fluorescent image, the image for display.

Further, the image processing means may perform spectral image processing on at least one of the ordinary image, the fluorescent image and the combined image.

Further, a selection means that can select whether the spectral image processing is performed on at least one of the ordinary image, the fluorescent image and the combined image may be further provided.

In the second and third fluorescent image obtainment apparatuses, the expression "illuminates substantially the same position of a region to be observed" means that the illumination range (area) with the illumination light and the illumination range (area) with the excitation light are at least partially overlap with each other.

A fourth fluorescent image obtainment apparatus of the present invention is a fluorescent image obtainment apparatus comprising:

a light illumination means that illuminates a region to be observed with illumination light and excitation light at the same time;

an imaging means that captures an image composed of light reflected from a region to be observed by illumination with the illumination light, and fluorescence output from the region to be observed by illumination with the excitation light;

a storage unit that stores an estimation matrix for calculating estimated spectral data; and

an image processing means that calculates estimated spectral data of a specific fluorescence wavelength band for each pixel of an image signal that has been output from the imaging means, the specific fluorescence wavelength band including at least an approximate center wavelength band of the fluorescence and the estimated spectral data being calculated based an image signal value of each of the pixels and the estimation matrix, and obtains, based on the estimated spectral data of the specific fluorescence wavelength band, information that reflects the radiation intensity of the fluorescence output from the region to be observed, and produces a fluorescent image based on the information that reflects the radiation intensity of the fluorescence.

In the fourth fluorescent image obtainment apparatus of the present invention, the "estimation matrix for calculating estimated spectral data" may take the spectral characteristic of illumination light and the spectral characteristic of the imaging means into consideration. Further, the estimation matrix may be used to calculate estimated spectral data including estimated spectral reflectance information about the region to be observed by performing matrix operation using the image signal. Further, the expression "specific fluorescence wavelength band including at least an approximate center wavelength band of the fluorescence" means a wavelength band including at least the center wavelength of the fluorescence or a wavelength in the vicinity of the center wavelength of the fluorescence. The specific fluorescence wavelength band should substantially reflect the intensity of the fluorescence. Further, the "information that reflects the radiation intensity of the fluorescence output from the region to be observed" should reflect at least whether the radiation intensity of fluorescence output from the region to be observed is high or low (the value of the intensity or the like).

In the fourth fluorescent image obtainment apparatus of the present invention, the light illumination means may illuminate the region to be observed with reference light that has a wavelength different from that of the excitation light at the same time as illuminating the region to be observed with the excitation light. Further, the imaging means may capture an image including reflection light of the reference light, the reflection light having been reflected from the region to be observed. Further, the image processing means may calculate reference light intensity that is the intensity of reflection light of the reference light, the reflection light being included in the image captured by the imaging means, and calculate, based on the estimated spectral data of the specific fluorescence wavelength band, pseudo-fluorescence intensity, which is the intensity of light in the specific fluorescence wavelength band, and calculate a pseudo-fluorescence yield, as radiation intensity information about the fluorescence, by dividing the pseudo-fluorescence intensity by the reference light intensity.

In the fourth fluorescent image obtainment apparatus of the present invention, the "reference light" may be light in a wavelength band that is different from the wavelength band of the illumination light. For example, the reference light may be IR light (infrared rays) or the like. The wavelength band of the reference light may be included in the wavelength band of the illumination light. When the wavelength band of the reference light is included in the wavelength band of the illumination light, the whole illumination light or light in a part of the wavelength band of the illumination light may be used as the reference light.

Further, the image processing means may obtain, for each pixel of the image signal output from the imaging means, estimated spectral data of a semi-ordinary wavelength band that does not include the specific fluorescence wavelength band by using the image signal value of each of the pixels and the estimation matrix, and produce a pseudo-ordinary image based on the estimated spectral data of the semi-ordinary wavelength band.

Here, the "semi-ordinary wavelength band" may be the wavelength band of the illumination light excluding a specific fluorescence wavelength band. Alternatively, the "semi-ordinary wavelength band" may be a part of the wavelength band of the illumination light. Further, the "pseudo ordinary image" may be either a color image or a black-and-white image (a gray-scale image).

Further, a display processing means that produces a fluorescence superimposed image in which the fluorescent image is superimposed on the pseudo-ordinary image may be provided.

Further, an input means that sets the specific fluorescence wavelength band by an input operation may be provided.

A fluorescence endoscope apparatus of the present invention is a fluorescence endoscope apparatus comprising:

an illumination light unit that includes a light source for illumination light that outputs the illumination light;
an excitation light unit that is detachably connected to the illumination light unit and that includes a light source for excitation light that outputs the excitation light;
a first light guide means that guides the illumination light or the excitation light to illuminate a region to be observed with the illumination light or the excitation light;
an imaging means that captures an image composed of light reflected from the region to be observed by illumination with the illumination light or an image composed of fluorescence output from the region to be observed by illumination with the excitation light;
an image processing means that produces an ordinary image based on the image composed of the reflection light, the image having been captured by the imaging means, and that produces a fluorescent image based on the image composed of the fluorescence, the image having been captured by the imaging means;
a detection means that detects whether the excitation light unit is connected to the illumination light unit; and
a light-output prevention means that prevents the excitation light unit from outputting the excitation light when the detection means has not detected that the excitation light unit is connected to the illumination light unit.

In the fluorescence endoscope apparatus of the present invention, the illumination light and the excitation light may be output at the same time. Alternatively, the illumination light and the excitation light may be output in time division. Further, the first light guide means may guide one of the illumination light and the excitation light. Alternatively, the first light guide means may guide both of the illumination light and the excitation light. Further, the imaging means may separately obtain an image composed of light reflected from a region to be observed that has been illuminated with illumination light and an image composed of fluorescence output from the region to be observed that has been illuminated with excitation light. Alternatively, the imaging means may obtain an image in which an image composed of reflection light and an image composed of fluorescence are superimposed one on the other.

In the fluorescence endoscope apparatus of the present invention, the illumination light unit may include a second light guide means that guides the excitation light. Further, the excitation light unit may include a third light guide means that extends to the outside of the excitation light unit and that guides the excitation light. Further, the detection means may detect whether the second light guide means and the third light guide means are optically connected to each other.

Further, the excitation light unit may include a drive unit for excitation light that drives the light source for excitation light. When the light-output prevention means prevents the excitation light unit from outputting the excitation light, the light-output prevention means may prevent the drive unit for excitation light and the light source for excitation light from being electrically connected to each other.

Further, the illumination light unit may include a drive unit for illumination light that drives the light source for illumination light and an illumination light control unit that controls the drive unit for illumination light. Further, the excitation light unit may include a drive unit for excitation light that drives the light source for excitation light and an excitation light control unit that controls the drive unit for excitation light. Further, the detection means may further detect whether the illumination light control unit and the excitation light control unit are electrically connected to each other.

Further, an operation may be performed in an ordinary image, mode or in a fluorescent image mode. In the ordinary image mode, the illumination light is output and the ordinary image is produced. In the fluorescent image mode, the excitation light is output and the fluorescent image is produced. Further, when the detection means has not detected that the excitation light unit is connected to the illumination light unit, the operation in the fluorescent image mode is not performed.

Further, an excitation light unit of the present invention may be an excitation light unit detachably connected to an illumination light unit of an endoscope apparatus, wherein the endoscope apparatus includes:

the illumination light unit that has a light source for illumination light that outputs illumination light;

a first light guide means that guides the illumination light or excitation light to illuminate a region to be observed with the illumination light or the excitation light;

an imaging means that captures an image of the region to be observed that has been illuminated with the illumination light or a fluorescence image of the region to be observed that has been illuminated with the excitation light; and

an image processing means that produces an ordinary image based on the image of the region to be observed, the image having been captured by the imaging means, and that produces a fluorescent image based on the fluorescence image of the region to be observed, the fluorescence image having been captured by the imaging means,

the excitation light unit comprising:

a light source for excitation light that outputs the excitation light;

a detection means that detects whether the excitation light unit is connected to the illumination light unit; and

a light-output prevention means that prevents the excitation light unit from outputting the excitation light to the outside of the excitation light unit when the detection means has detected that the excitation light unit is not connected to the illumination light unit.

In the first through fourth fluorescent image obtainment apparatuses and fluorescence endoscope apparatuses of the present invention, the "illumination light" may be, for example, white light of continuous wavelengths. Alternatively, the "illumination light" may be white light obtained by outputting R (red) light, G (green) light and B (blue) light at the same time.

According to the first fluorescent image obtainment method and apparatus of the present invention, when an image composed of light reflected from a region to be observed, the region having been illuminated with illumination light, and an image composed of fluorescence output from the region to be observed, the region having been illuminated with excitation light at the same time as illumination with the illumination light, are captured, and an ordinary image is produced based on the captured image composed of the reflection light, and a fluorescent image is produced based on the captured image composed of fluorescence, the light amount of the illumination light is controlled so that a representative luminance value of the ordinary image becomes a predetermined luminance value, and the light amount of the excitation light is controlled so that the ratio of the light amount of the excitation light to the light amount of the illumination light becomes a predetermined ratio. Therefore, it is possible to prevent the risk of increasing the light amount of the excitation light when the distance between the excitation light output end and the region to be observed is short, in other words, when the light amount of the excitation light should not be increased. Therefore, it is possible to obtain a fluorescent image that reflects the tissue characteristic of the region to be observed. Hence, it is possible to improve the reliability of the fluorescent image.

Further, according to the second fluorescent image obtainment method and apparatus of the present invention, a white light source means is driven so as to continuously output white light, and an excitation light source means is pulse-driven based on a pulse signal so as to output pulsed excitation light. Further, an image composed of light reflected from a region to be observed by illumination with the illumination light, and an image including fluorescence output from the region to be observed by illumination with the excitation light are captured. An ordinary image is produced based on the captured image composed of the reflection light, and a fluorescent image is produced based on the captured image including fluorescence. Further, the light amount of the illumination light is controlled so that a representative luminance value of the ordinary image becomes a predetermined luminance value, and the light amount of the excitation light is controlled by controlling the pulse signal so that the ratio of the light amount of the excitation light to the light amount of the illumination light becomes a predetermine ratio. Therefore, it is possible to constantly control the ratio of the light amount of the excitation light to the light amount of the illumination light at a constant value. Further, it is possible to constantly maintain the good balance between the lightness of the ordinary image and the lightness of the fluorescent image. Further, since the light amount of the excitation light is controlled by digitally controlling the pulse signal, it is possible to improve the noise resistance. Further, since pulse driving is performed, it is possible to increase the light amount when the excitation light is output. Hence, it is possible to contribute to reduction of the cost of the light source.

Further, in the first and second fluorescent image obtainment methods and apparatuses of the present invention, when a setting means that sets the ratio of the light amount of the excitation light to the light amount of the illumination light by an input operation is provided, the ratio of the light amount of the excitation light to the light amount of the illumination light can be arbitrarily set. Therefore, it is possible to set a ratio that is appropriate for the purpose of diagnosis or the like. Hence, it is possible to contribute to highly accurate screening.

Further, according to the third fluorescent image obtainment method and apparatus of the present invention, an illumination light source means is driven so as to continuously output illumination light, and an excitation light source means is pulse-driven so as to output pulsed excitation light. Further, a region to be observed is continuously illuminated with the illumination light, and a region that is substantially the same as the region illuminated with the illumination light is illuminated with the pulsed excitation light. Further, a first image composed of light reflected from the region to be observed is captured in a time period when the region to be observed is illuminated only with the illumination light in the process of continuously illuminating the region to be observed with the illumination light and illuminating substantially the same region with the pulsed excitation light, and a second image composed of light reflected from the region to be observed and fluorescence output from the region to be observed is captured in a time period when the region to be observed is illuminated with both of the illumination light and the excitation light in the process of continuously illuminating the region to be observed with the illumination light and illuminating substantially the same region with the pulsed excitation light. Further, an ordinary image based on the first image is produced, and a fluorescent image based on the first image and the second image is produced. Therefore, it is possible to produce the ordinary image and the fluorescent image without providing a mechanical structure, such as a spectral structure. Hence, it is possible to simplify the whole apparatus and to reduce the cost of the apparatus.

Further, in the third fluorescent image obtainment apparatus of the present invention, when an image for display is produced by performing an operation by using at least two of the ordinary image, the fluorescent image and the combined image or when spectral image processing is performed on at least one of the ordinary image, the fluorescent image and the combined image, it is possible to produce an image for display that can make it possible to perform more accurate screening.

When a selection means that can select whether the spectral image processing is performed on at least one of the ordinary image, the fluorescent image and the combined image is further provided, and the spectral image processing is performed, if the selection means selects performance of the spectral image processing, it is possible to produce an image for display that is appropriate for the purpose of the image.

Further, according to the fourth fluorescent image obtainment method and apparatus of the present invention, an image composed of light reflected from a region to be observed, the region having been illuminated with illumination light, and fluorescence output from the region to be observed, the region having been illuminated with excitation light at the same time as illumination with the illumination light, is captured. Further, estimated spectral data of a specific fluorescence wavelength band for each pixel of image signals of the captured image, the specific fluorescence wavelength band including at least an approximate center wavelength band of the fluorescence, is calculated based an image signal value of each of the pixels and an estimation matrix for calculating the estimated spectral data, and the estimation matrix having been stored in advance. Further, information that reflects the radiation intensity of the fluorescence output from the region to be observed is obtained based on the estimated spectral data of the specific fluorescence wavelength band, and a fluorescent image is produced based on the information that reflects the radiation intensity of the fluorescence. Therefore, it is possible to prevent the number of obtainable frames of the fluorescent image per unit time from decreasing. Hence, even when the fluorescent image is displayed as a motion image, it is possible to produce an efficient image for display.

Further, the fluorescence endoscope apparatus of the present invention includes an illumination light unit that includes a light source for illumination light that outputs the illumination light, and an excitation light unit that is detachably connected to the illumination light unit and that includes a light source for excitation light that outputs the excitation light. Further, the fluorescence endoscope apparatus includes a first light guide means that guides the illumination light or the excitation light to illuminate a region to be observed with the illumination light or the excitation light, and an imaging means that captures an image composed of light reflected from the region to be observed by illumination with the illumination light or an image composed of fluorescence output from the region to be observed by illumination with the excitation light. Further, the fluorescence endoscope apparatus includes an image processing means that produces an ordinary image based on the image composed of the reflection light, the image having been captured by the imaging means, and that produces a fluorescent image based on the image composed of the fluorescence, the image having been captured by the imaging means, and a detection means that detects whether the excitation light unit is connected to the illumination light unit. Further, the fluorescence endoscope apparatus includes a light-output prevention means that prevents the excitation light unit from outputting the excitation light when the detection means has not detected that the excitation light unit is connected to the illumination light unit. Therefore, when the excitation light unit is not connected to the illumination light unit, the excitation light is not output from the excitation light unit. Hence, even if the user of the fluorescence endoscope apparatus is not familiar with or not used to handling of the light source for excitation light, such as high-luminance LED or laser, it is possible to safely obtain the fluorescent image.

Further, the excitation light unit of the present invention is an excitation light unit detachably connected to an illumination light unit of an endoscope apparatus that includes:

the illumination light unit that has a light source for illumination light that outputs illumination light;
a first light guide means that guides the illumination light or excitation light to illuminate a region to be observed with the illumination light or the excitation light;
an imaging means that captures an image of the region, to be observed that has been illuminated with the illumination light or a fluorescence image of the region to be observed that has been illuminated with the excitation light; and

an image processing means that produces an ordinary image based on the image of the region to be observed, the image having been captured by the imaging means, and that produces a fluorescent image based on the fluorescence image of the region to be observed, the fluorescence image having been captured by the imaging means. Further, the excitation light unit includes:

a light source for excitation light that outputs the excitation light;
a detection means that detects whether the excitation light unit is connected to the illumination light unit; and
a light-output prevention means that prevents the excitation light unit from outputting the excitation light to the outside of the excitation light unit when the detection means has detected that the excitation light unit is not connected to the illumination light unit. Therefore, when the excitation light unit is not connected to the illumination light unit, the excitation light is not output from the excitation light unit. Hence, even if the user of the fluorescence endoscope apparatus is not familiar with or not used to handling of the light source for excitation light, such as high-luminance LED or laser, it is possible to safely handle the excitation light unit.

BRIEF DESCRIPTION OF THE DRAWINGS

Figure 1 is a block diagram illustrating the configuration of a fluorescence endoscope apparatus to which a first embodiment of the present invention has been applied;
Figure 2A is a diagram illustrating the relation between wavelengths and pseudo-spectral reflectance data;
Figure 2B is a diagram illustrating the relation between wavelengths and spectral reflectance data;

Figure 3 is a diagram illustrating the relation between wavelengths in a specific fluorescence wavelength band and pseudo-spectral reflectance data;
Figure 4 is a diagram illustrating the relation between wavelengths in a semi-ordinary wavelength band and pseudo-spectral reflectance data;
Figure 5 is a diagram for explaining a method for assigning color to a fluorescence yield;
Figure 6 is a diagram for explaining another method for assigning color to a fluorescence yield;
Figure 7 is a block diagram illustrating the configuration of a modified example of the fluorescence endoscope apparatus to which the first embodiment of the present invention has been applied;
Figure 8 is a block diagram illustrating the configuration of a fluorescence endoscope apparatus to which a second embodiment of the present invention has been applied;
Figure 9 is a diagram illustrating an example of light output pattern of illumination light and excitation light in the fluorescence endoscope apparatus in the second embodiment of the present invention;
Figure 10 is a diagram illustrating another example of light output pattern of illumination light and excitation light in the fluorescence endoscope apparatus of the second embodiment of the present invention;
Figure 11 is a block diagram illustrating the configuration of a modified example of the fluorescence endoscope apparatus to which the second embodiment of the present invention has been applied;
Figure 12 is a block diagram illustrating the configuration of a fluorescence endoscope apparatus to which a third embodiment of the present invention has been applied;
Figure 13 is a diagram illustrating an example of light output pattern of illumination light and excitation light in the fluorescence endoscope apparatus of the third embodiment of the present invention;
Figure 14 is a diagram illustrating the configuration of a modified example of the fluorescence endoscope apparatus to which the third embodiment of the present invention has been applied; and
Figure 15 is a diagram for explaining the spectrum of fluorescence output from normal tissue and lesion tissue.

DESCRIPTION OF THE PREFERRED EMBODIMENTS

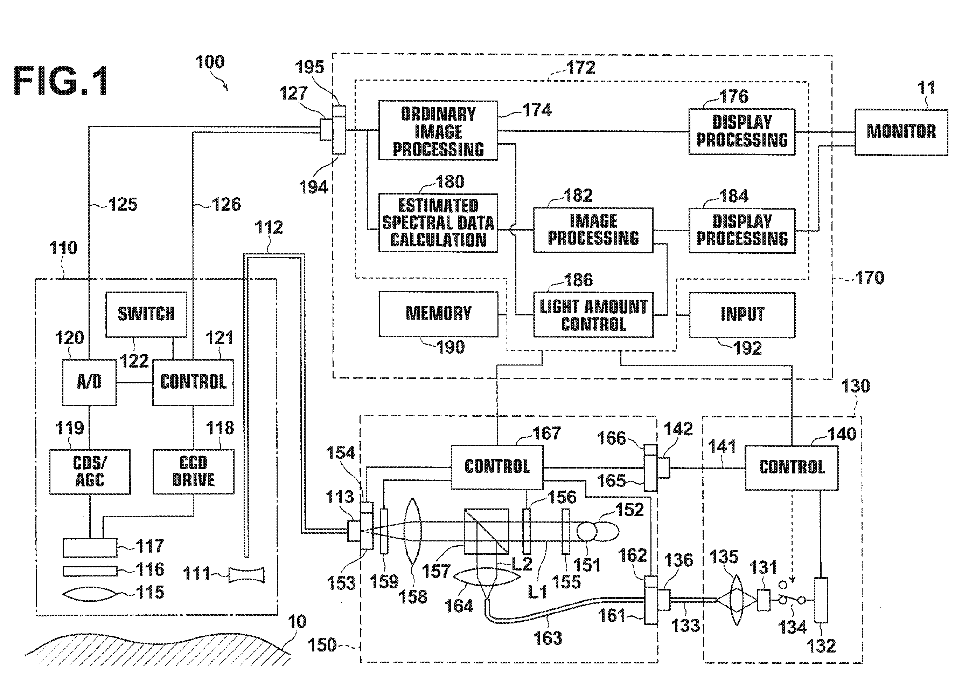

Hereinafter, with reference to drawings, a first embodiment of the present invention will be described in detail. Figure 1 is a schematic diagram illustrating the configuration of a fluorescence endoscope apparatus to which a first embodiment of the present invention has been applied.

A fluorescence endoscope apparatus 100 according to the present embodiment operates in an ordinary image mode and in a fluorescent image mode. In the ordinary image mode, a region 10 to be observed is illuminated with illumination light L1 to obtain color ordinary images and the obtained images are displayed as a motion image (a video image, a dynamic image or the like) . In the fluorescent image mode, the region 10 to be observed is illuminated with the illumination light L1 and excitation light L2 to obtain color images, and pseudo-color ordinary images are obtained from the obtained color images by operation processing, which will be described later. Further, the pseudo-color ordinary images and fluorescence superimposed images are displayed as a motion image. As illustrated in Figure 1, the fluorescence endoscope apparatus 100 includes a scope unit 110, a processor unit 170, an illumination light unit 150, and an excitation light unit 130. The scope unit 110 is inserted into the body cavity of a person to be examined (a subject to be examined or a patient) to observe the region 10 to be observed. The processor unit 170 is electrically connected to the scope unit 110 in a detachable manner. The illumination light unit 150 is optically connected to the scope unit 110 in a detachable manner, and stores a xenon lamp 151 that outputs the illumination light L1. The excitation light unit 130 is both electrically and optically connected to the illumination light unit 150 in a detachable manner, and stores a GaN-based semiconductor laser 131 that outputs the excitation light L2. The processor unit 170 and the illumination light unit 150 may be structured as a single body or as separate bodies.

Further, an optical system 111 for illumination is provided at a leading end of the scope unit 110. The optical system 111 for illumination faces an end of a light guide 112 that guides the illumination light L1. The light guide 112 extends to the outside of the scope unit 110, and an optical connector 113 is provided at the other end of the light guide 112 to be detachably connected to an optical connector 153 of the illumination light unit 150, which will be described later.

Further, at a leading end of the scope unit 110, an imaging lens 115, an excitation light cut filter 116, and a CCD (charge coupled device) 117, which is a solid-state imaging device, are coaxially provided in this order. The imaging lens 115 forms an image of the region 10 to be observed on the CCD 117. Further, as the excitation light cut filter 116, a filter that blocks only the excitation light and transmits light of the other wavelengths, such as a notch filter that blocks light only in an extremely narrow band for example, may be used. Further, a primary-color-type mosaic filter including an RGB color filter is attached to the imaging surface of the CCD 117. A CCD drive circuit 118 and a CDS/AGC (correlated double sampling / automatic gain control) circuit 119 are connected to the CCD 117. The CCD drive circuit 118 forms a drive pulse based on a synchronized signal, and the CDS/AGC circuit 119 samples and amplifies an image (video) signal output from the CCD 117. Further, an A/D (analog to digital) converter 120 that digitizes an analog output from the CDS/AGC circuit 119 is connected to the CDS/AGC circuit 119. Further, in the scope unit 110, a control unit 121 is provided. The control unit 121 controls each circuit provided in the scope unit 110, and controls communication between the processor unit 170 and the scope unit 110. Further, a press-type switch 122 is connected to the control unit 121 and provided in the vicinity of the base portion (portion close to the user who handles the scope unit 110) of the scope unit 110. The press-type switch 122 is used to switch the operation modes. Further, an end of a signal line 125 is connected to the A/D converter 120, and an end of a signal line 126 is connected to the control unit 121. The signal line 125 and the signal line 126 extend to the outside of the main body of the scope unit 110, and a connector 127 is connected to the other end of the signal line 125 and the other line of the signal line 126. The connector 127 is detachably connected to a connector 194 of the processor unit 170, which will be described later.

The illumination light unit 150 includes a xenon lamp 151 that outputs the illumination light L1, a drive circuit 152 that drives the xenon lamp 151, and an optical connector 153. The optical connector 153 is detachably connected to the optical connector 113 that is provided at an end of the light guide 112 of the scope unit 110. Further, a connection detection unit 154 that detects whether the optical connector 153 is connected to the optical connector 113 is provided for the optical connector 153. Further, a wavelength filer 155, a diaphragm (an aperture diaphragm) 156, a dichroic mirror 157, a condensing lens 158, and a rotary shutter 159 are arranged between the xenon lamp 151 and the optical connector 153. The wavelength filer 155 limits the wavelength band of the illumination light L1 to a wavelength band that is longer than or equal to 410 nm and shorter than or equal to 700 nm. The diaphragm 156 controls the light amount of the illumination light L1, and the dichroic mirror 157 transmits light that has a wavelength of longer than or equal to 410 nm and reflects light that has a wavelength shorter than 410 nm at a right angle. Further, the illumination light unit 150 includes an optical connector 161 that is detachably connected to an optical connector 136 that is provided at a leading end of a light guide 133 of the excitation light unit 130, which will be described later. Further, a connection detection unit 162 that detects whether the optical connector 161 is connected to the optical connector 136 is provided for the optical connector 161. The optical connector 161 is connected to an end (light incident end) of a light guide 163 that guides the excitation light within the illumination light unit 150. Further, the other end (light output end) of the light guide 163 is placed at a position so that the excitation light L2 output from the light guide 163 enters the dichroic mirror 157. Further, a lens 164 is provided between the light output end of the light guide 163 and the dichroic mirror 157.

Further, the illumination light unit 150 includes a connector 165 that is detachably connected to a connector 142 of the excitation light unit 130, which will be described alter. The connector 165 has a connection detection unit 166 that detects whether the optical connector 165 is connected to the optical connector 142. Further, the illumination light unit 150 includes a control unit 167 that is connected to each element, such as the connector 165 and the connection detection unit 166, provided in the illumination light unit 150, and that controls each of the elements. Further, the control unit 167 controls communication with the processor unit 170 and the excitation light unit 130.

The excitation light unit 130 includes a GaN-based semiconductor laser 131 that outputs excitation light L2, a drive circuit 132 that drives the semiconductor laser 131, and a light guide 133 that guides the excitation light L2 output from the semiconductor laser 131. The light guide 133 extends from the case of the excitation light unit 130, and the other end of the light guide 133 is connected to the optical connector 136. The optical connector 136 is detachably connected to the optical connector 161 of the illumination light unit 150. A switch 134 is provided between the semiconductor laser 131 and the drive circuit 132, and a light-condensing optical system 135 is provided between the semiconductor laser 131 and an end (light incident end) of the light guide 133.

Further, the excitation light unit 130 includes a control unit 140 that is connected to each element, such as the drive circuit 132 and the switch 134, provided in the excitation light unit 130, and that controls each of the elements. Further, the control unit 140 controls communication between the illumination light unit 150 and the excitation light unit 130. The control unit 140 is connected to an end of a signal line 141. The signal line 141 extends to the outside of the case of the excitation light unit 130, and a connector 142 is provided at the other end of the signal line 141. The connector 142 is detachably connected to the connector 165 of the illumination light unit 150.

Meanwhile, a processing unit 172 is provided in the processor unit 170. The processing unit 172 includes an ordinary image processing unit 174 and a display processing unit 176 that perform signal processing when an ordinary image mode is selected. Further, the processing unit 172 includes an estimated spectral data calculation unit 180, an image processing unit 182 and a display processing unit 184 that perform image processing when a fluorescent image mode is selected. Further, the processing unit 172 includes a light amount control unit 186 that controls the intensity of the illumination light and the excitation light.

When the ordinary image mode is selected, the ordinary image processing unit 174 performs various kinds of image processing on three-color image signals of R, G and B output from the A/D converter 120 of the scope unit 110, and generates a Y/C signal composed of a luminance (Y) signal and chrominance [C (R - Y and B - Y)] signals. Further, the ordinary image processing unit 174 outputs the Y/C signal to the display processing unit 176. The display processing unit 176 performs various kinds of signal processing on the Y/C signal to generate color ordinary image signal for display. The color ordinary image signal is output to a monitor 11, such as a liquid crystal display and a CRT, for example.

When the fluorescent image mode is selected, the estimated spectral data calculation unit 180 calculates estimated spectral data for each pixel by using the three color image signal of R, G and B output from the A/D converter 120 of the scope unit 110 and estimation matrix data for calculating the spectral data, and the estimation matrix data having been stored in advance. Further, the estimated spectral data calculation unit 180 outputs the estimated spectral data to the image processing unit 182.

In the image processing unit 182, first, estimated spectral data for a specific fluorescence wavelength band that includes the wavelength of 480 nm, which is the center wavelength of the fluorescence output from the region 10 to be observed when the region 10 is illuminated with the excitation light L2, is obtained. For example, the specific fluorescence wavelength band is a band of 460 nm to 500 nm. Further, a fluorescent image is produced based on the estimated spectral data for the specific fluorescence wavelength band. Further, estimated spectral data for a semi-ordinary wavelength band that does not include the specific fluorescence wavelength band is obtained. For example, the semi-ordinary wavelength band is a band of 410 nm to 460 nm and a band of 500 nm to 700 nm. Further, pseudo-color ordinary image data and pseudo-black-and-white ordinary image are produced based on the estimated spectral data for the semi-ordinary wavelength band. Further, a fluorescence superimposed image data is generated by superimposing the fluorescent image data on the pseudo-black-and-white ordinary image data. The pseudo-color ordinary image data and the fluorescence superimposed image data are output to the display processing unit 184. In the display processing unit 184, a display image that displays the pseudo-color ordinary image data and the fluorescence superimposed image data next to each other is produced, or a color image signal for display in which the pseudo-color ordinary image data and the fluorescence superimposed image data are synthesized (combined) into a single image is produced, and output to the monitor 11.

The light amount control unit 186 is connected to the ordinary image processing unit 174 and the image processing unit 182. When an illumination ordinary image mode is selected, the light amount control unit 186 controls the light amount of the illumination light L1 based on the luminance of the color ordinary image. When a fluorescent image mode is selected, the light amount control unit 186 controls the light amount of the illumination light L1 and the light amount of the excitation light L2 based on the luminance of the pseudo-color ordinary image.

Further, the processing unit 172 is connected to a memory 190, a keyboard-type input unit 192 and a connector 194. The connector 194 is detachably connected to the connector 127 of the scope unit 110. The connector 194 has a connection detection unit 195 that detects whether the connector 127 is connected to the connector 194. Further, the processing unit 172 is connected to the control unit 121 of the scope unit 110, the control unit 167 of the illumination light unit 150, and the control unit 140 of the excitation light unit 130.

In the memory 190, estimation matrix data for calculating the estimated spectral data of the region 10 to be observed is stored. The estimation matrix data is stored, as a table, in the memory 190 in advance. The estimation matrix data takes the spectral characteristic of the illumination light L1 and the spectral characteristic of the whole imaging system including the color sensitivity characteristics of the imaging device, the transmissivity of the color filter or the like, into consideration. The spectral data of the region 10 to be observed is obtained by performing an operation using the RGB image signals obtained by imaging by the CCD 117 and the estimation matrix data. Accordingly, the spectral data of the region 10 to be observed that depends on none of the type of the illumination light, the unique spectral characteristic of the imaging system and the like can be obtained. The estimation matrix data is disclosed in Japanese Unexamined Patent Publication No. 2003-093336 and U.S. Patent Application Publication No. 20070183162 or the like in detail. The following Table 1 shows an example of the estimation matrix data stored in the memory 190 in the present embodiment:

Table 1

| PARAMETER | $k_{pr}$ | $k_{pg}$ | $k_{pb}$ |
|---|---|---|---|
| p1 | $k_{1r}$ | $k_{1g}$ | $k_{1b}$ |
| ⋮ | ⋮ | ⋮ | ⋮ |
| p18 | $k_{18r}$ | $k_{18g}$ | $k_{18b}$ |
| p19 | $k_{19r}$ | $k_{19g}$ | $k_{19b}$ |
| p20 | $k_{20r}$ | $k_{20g}$ | $k_{20b}$ |
| p21 | $k_{21r}$ | $k_{21g}$ | $k_{21b}$ |
| p22 | $k_{22r}$ | $k_{22g}$ | $k_{22b}$ |
| p23 | $k_{23r}$ | $k_{23g}$ | $k_{23b}$ |
| ⋮ | ⋮ | ⋮ | ⋮ |
| p43 | $k_{43r}$ | $k_{43g}$ | $k_{43b}$ |
| p44 | $k_{44r}$ | $k_{44g}$ | $k_{44b}$ |
| p45 | $k_{45r}$ | $k_{45g}$ | $k_{45b}$ |
| p46 | $k_{46r}$ | $k_{46g}$ | $k_{46b}$ |
| p47 | $k_{47r}$ | $k_{47g}$ | $k_{47b}$ |
| p48 | $k_{48r}$ | $k_{48g}$ | $k_{48b}$ |
| p49 | $k_{49r}$ | $k_{49g}$ | $k_{49b}$ |
| p50 | $k_{50r}$ | $k_{50g}$ | $k_{50b}$ |

(continued)

| PARAMETER | $k_{pr}$ | $k_{pg}$ | $k_{pb}$ |
|---|---|---|---|
| p51 | $k_{51r}$ | $k_{51g}$ | $k_{51b}$ |
| p52 | $k_{52r}$ | $k_{52g}$ | $k_{52b}$ |
| ⋮ | ⋮ | ⋮ | ⋮ |
| p59 | $k_{59r}$ | $k_{59g}$ | $k_{59b}$ |

The matrix data in Table 1 includes 59 wavelength band parameters (coefficient sets) p1 through p59. For example, the wavelength band of 410 nm to 700 nm is divided into 59 at intervals of 5nm. Each of the parameters p1 through p59 includes coefficients $K_{pr}$, $K_{pg}$, $K_{pb}$ (p = 1 to 59) for matrix operation.

Next, the operation of the fluorescence endoscope apparatus according to the present invention that is structured as described above will be described. First, an operation in the ordinary image mode will be described. In the ordinary image mode, the region 10 to be observed is illuminated with the illumination light L1 to obtain color ordinary images, and the color ordinary images are displayed as a motion image.

Before the fluorescence endoscope apparatus of the present embodiment is used, a washed and sterilized scope unit 110 is attached to the processor unit 170 and the illumination light unit 150. Further, the connector 127 connected to an end of the signal line 125 of the scope unit 110 and an end of the signal line 126 of the scope unit 110 is connected to the connector 194 of the processor unit 170. Further, the optical connector 113 provided at the leading end of the light guide 112 is connected to the optical connector 153 of the illumination light unit 150. When the connector 127 is connected to the connector 194, the connection detection unit 195 provided for the connector 194 sends a connection signal to the processing unit 172. Further, when the optical connector 113 is connected to the optical connector 153, the connection detection unit 154 provided for the optical connector 153 outputs a connection signal to the control unit 167.

When the processing unit 172 receives connection signals from the connection detection unit 195 and the connection detection unit 154, a rotary shutter 159 of the illumination light unit 150 is rotated so that an operation in the ordinary image mode can be performed. Further, the function mode of a predetermined key of the input unit 192 is set through the processing unit 172 of the processor unit 170. Further, the function mode of the switch 122 is set through the processing unit 172 and the control unit 121 of the scope unit 110. Since the processing unit 172 controls processing, the operation mode switches between a stop state and an ordinary image mode when the user presses a predetermined key of the input unit 192 or the switch 122.

When the user presses the predetermined key of the input unit 192 or the switch 122 once, an operation in the ordinary image mode starts. In the illumination light unit 150, the xenon lamp 151 is turned on by the drive circuit 152, and the illumination light L1 is output. The illumination light L1 passes through the wavelength filter 155, the diaphragm 156 and the dichroic mirror 157 and condensed onto the end surface of the optical connector 113 by the condensing lens 158. The condensed light enters the light guide 112. The illumination light L1 propagates through the light guide 112, and is output from the leading end of the light guide 112. The output light is output to the region 10 to be observed through the optical system 111 for illumination, and illuminates the region 10 to be observed.

The wavelength band of the illumination light L1 is limited to greater than or equal to 410 nm and less than or equal to 700 nm by the wavelength filter 155. The light amount of the illumination light L1 is controlled by the diaphragm 156. The light amount control operation of the illumination light L1 by the diaphragm 156 will be described later.

The CCD 117 that is driven by the CCD drive circuit 118 captures an image of the region 10 to be observed, and outputs an imaging signal. The imaging signal is amplified in the CDS/AGC circuit 119 by correlated double sampling and automatic gain control. Further, A/D conversion is performed on the amplified imaging signal in the A/D converter 120, and output as an RGB color image to the ordinary image processing unit 174 in the processing unit 172 of the processor unit 170. In the ordinary image processing unit 174, when the ordinary image mode is selected, various kinds of signal processing is performed on the three color image signal of R, G and B that has been output from the A/D converter 120 of the scope unit 110. After the image processing, a Y/C signal (color ordinary image signal) that is composed of luminance signal Y and chrominance signals C is generated, and output to the display processing unit 176. In the display processing unit 176, various kinds of signal processing, such as I/P conversion and noise removal processing, is performed on the Y/C signal, and the processed signal is output to the monitor 11.

Further, the ordinary image processing unit 174 outputs the luminance signal Y for each pixel or an average luminance signal Y' of a plurality of adjacent pixels to the light amount control unit 186. The light amount control unit 186 calculates an average luminance value Ya of pixels in a specified area for each frame, and compares the average luminance value Ya with a reference luminance value Yr (standard luminance value) that has been stored in the memory 190 in advance. Further, the light amount control unit 186 selects a diaphragm control signal based on the result of comparison, and outputs the diaphragm control signal to the control unit 167 of the illumination light unit 150. As the diaphragm control signal, when the average luminance value Ya is greater than the reference luminance value Yr, a signal that reduces the aperture amount of the diaphragm 156 is selected, in other words, a signal that reduces the light passing through the aperture of the diaphragm 156 is selected. When the average luminance value Ya is smaller than the reference luminance value Yr, a signal that increase the aperture amount of the diaphragm 156 is selected, in other words, a signal that increases the light passing through the aperture of the diaphragm 156 is selected. When the average luminance value Ya and the reference luminance value Yr are substantially the same, a signal that maintains the same aperture amount is selected.

The control unit 167 of the illumination light unit 150 controls the aperture amount of the diaphragm 156 based on the diaphragm control signal.

Next, an operation in the fluorescent image mode will be described. Before the fluorescence endoscope apparatus is used in the fluorescent image mode, a washed and sterilized scope unit 110 is attached to the processor unit 170 and the illumination light unit 150. Further, the connector 127 connected to an end of the signal line 125 of the scope unit 110 and an end of the signal line 126 of the scope unit 110 is connected to the connector 194 of the processor unit 170. When the connector 194 is connected to the connector 127, the connection detection unit 195 provided for the connector 194 outputs a connection signal to the processing unit 172. Further, the optical connector 113 provided at the leading end of the light guide 112 is connected to the optical connector 153 of the illumination light unit 150. When the optical connector 153 is connected to the connector 113, the connection detection unit 154 provided for the optical connector 153 sends a connection signal to the control unit 167.

Further, the excitation light unit 130 is connected to the illumination light unit 150. The connector 142 provided at the leading end of the signal line 141 of the excitation light unit 130 is connected to the connector 165 of the illumination light unit 150. When the connector 142 is connected to the connector 165, the connection detection unit 166 provided for the connector 165 outputs a connection signal to the control unit 167. Further, the optical connector 136 provided at the leading end of the light guide 133 is connected to the optical connector 161 of the illumination light unit 150. When the optical connector 136 is connected to the optical connector 161, the connection detection unit 162 provided for the optical connector 161 outputs a connection signal to the control unit 167.

The control unit 140 of the excitation light unit 130 communicates with the control unit 167 of the illumination light unit 150. When the control unit 140 receives connection signals from the connection detection unit 166 and the connection detection unit 162, the control unit 140 closes (connects) the switch 134 of the excitation light unit 130 to electrically connect the semiconductor laser 131 and the drive circuit 132. Consequently, the semiconductor laser 131 becomes drivable by the drive circuit 132. Further, the function mode of a predetermined key of the input unit 192 is set through the processing unit 172 of the processor unit 170. Further, the function mode of the switch 122 is set through the processing unit 172 and the control unit 121 of the scope unit 110. Since the control unit 140 controls processing, the operation mode switches between a stop state, an ordinary image mode and a fluorescent image mode when the user presses a predetermined key of the input unit 192 or the switch 122. When the connection signals are not received from both of the connection detection unit 166 and the connection detection unit 162, in other words, when no connection signals are received from the two connection detection units, or no connection signal is received from one of the connection detection units, the switch 134 in the excitation light unit 130 is constantly open (disconnected). Hence, when the excitation light unit 130 is not connected to the illumination light unit 150, the semiconductor laser 131 is not driven.

Further, when the fluorescence endoscope apparatus is operating in the ordinary image mode, if the user (doctor or the like) presses the predetermined key of the input unit 192 or the switch 122 once, the operation in the fluorescent image mode starts.

The operation of the excitation light unit 130 starts in addition to the operation of the illumination light unit 150. The semiconductor laser 131 is driven by the drive circuit 132, and excitation light L2 that has a wavelength of 405 nm is output. The excitation light L2 is condensed by the light-condensing optical system 135, and enters the end surface of the light guide 133. The excitation light L2 propagates through the light guide 133, and enters the light guide 163 through the optical connector 136 and the optical connector 161. The excitation light L2 propagates through the light guide 163 and is output from an end of the light guide 163 . The output excitation light L2 is collimated by a collimator lens 164, and enters the dichroic mirror 157. Since the wavelength of the excitation light L2 is 405 nm, the excitation light L2 is reflected at a right angle from the dichroic mirror 157, and condensed onto the end surface of the optical connector 113 by the condensing lens 158. The condensed light enters the light guide 112. The excitation light L2 propagates through the light guide 112, and is output from the leading end of the light guide 112. Further, the excitation light L2 is output to the region 10 to be observed through the optical system 111 for illumination to illuminate the region 10 to be observed. In this case, the region 10 to be observed is illuminated with both of the illumination light L1 and the excitation light L2 at the same time. The light amount of the excitation light L2 is controlled by the drive current of the drive circuit 132. The light amount control operation of the excitation light L2 by the drive circuit will be described later.

The CCD 117 that is driven by the CCD drive circuit 118 captures an image composed of the reflection light of the illumination light L1, reflected from the region 10 to be observed, and fluorescence output from the region 10 to be observed, the fluorescence being output by illumination with the excitation light L2. Since an excitation light cut filter that cuts light that has a wavelength lower than or equal to 410 nm is provided at the leading end of the CCD 117, most of the reflection light of the excitation light L2 does not enter the CCD 117. The CCD 117 outputs imaging signals, and the imaging signals are amplified in the CDS/AGC circuit 119 by correlated double sampling and automatic gain control. Further, A/D conversion is performed on the amplified imaging signal in the A/D converter 120 and output as an RGB color image to the estimated spectral data calculation unit 180 in the processing unit 172 of the processor unit 170.

The estimated spectral data calculation unit 180 performs matrix operation represented by the following formula 1 for each pixel to generate estimated spectral data (q1 through q59) with respect to the three-color image signal of R, G and B, and outputs the generated estimated spectral data to the image processing unit 182. The estimated spectral data is generated by using a matrix of $3 \times 59$ including all of parameters of estimated matrix data stored in the memory 190.

[Formula 1]

$$
\begin{bmatrix} q_1 \\ q_2 \\ \vdots \\ q_{59} \end{bmatrix} = \begin{bmatrix} k_{1r} & k_{1g} & k_{1b} \\ k_{2r} & k_{2g} & k_{2b} \\ & \vdots & \\ k_{59r} & k_{59g} & k_{59b} \end{bmatrix} \times \begin{bmatrix} R \\ G \\ B \end{bmatrix}
$$

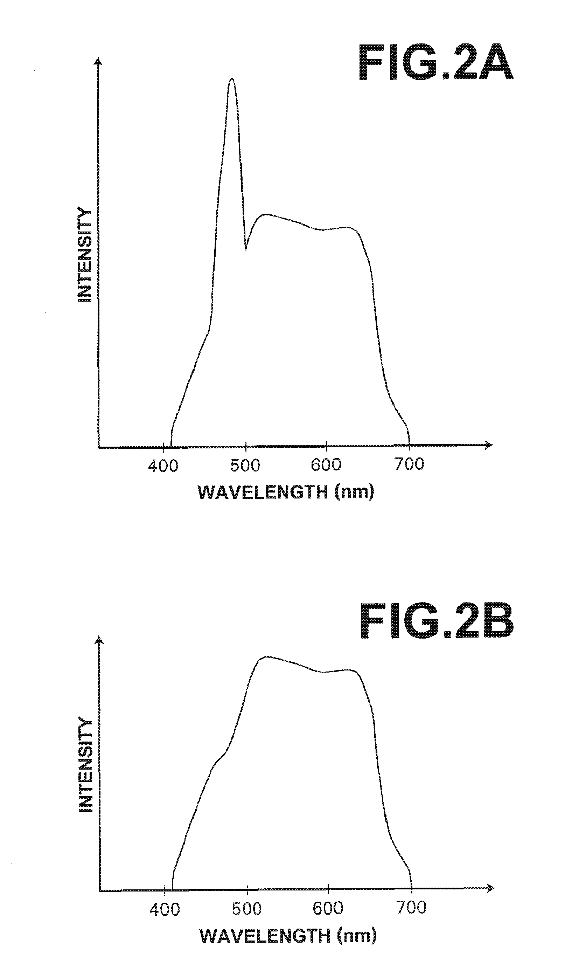

Figures 2A and 2B are diagrams illustrating examples of spectral distribution of the estimated spectral data (q1 through q59) generated for each pixel. Figure 2A illustrates a spectral distribution of pixels corresponding to the region 10 to be observed that is outputting fluorescence. Figure 2B illustrates a spectral distribution of pixels corresponding to the region 10 to be observed that is not outputting fluorescence. In each of Figures 2A and 2B, the horizontal axis represents the wavelength corresponding to each of data values q1 through q59 of the estimated spectral data, and the vertical axis represents the intensity of each of the data values q1 through q59.

As illustrated in Figure 2B, the spectral distribution obtained from the region 10 to be observed that is not outputting fluorescence reflects the spectral reflectance in the region 10 to be observed. Specifically, the intensity of each of the data values q1 through q59 reflects the product of the spectral reflectance of the region 10 to be observed and the intensity of light that enters each pixel of the CCD 117.

As illustrated in Figure 2A, the spectral distribution of the estimated spectral data (q1 through q59) obtained from the region 10 to be observed that is outputting fluorescence reflects the spectral reflectance in the region 10 to be observed and the intensity of fluorescence in the vicinity of the wavelength of 480 nm, which is the center wavelength of fluorescence. Specifically, the intensity of each of the data values q1 through q59 reflects the spectral reflectance of the region 10 to be observed, the spectral radiation rate of the output fluorescence, and the intensity of light that enters each pixel of the CCD 117. The estimation matrix that is used to generate the estimated spectral data (q1 through q59) is a matrix for calculating the spectral reflectance of the region 10 to be observed. Therefore, each of the data values q1 through q59 does not exactly reflect the spectral radiation rate of fluorescence, but includes information about the value of the spectral radiation rate of the fluorescence, i.e., whether the value of the spectral radiation rate is large or small, or the like. Therefore, it is possible to calculate the pseudo-fluorescence yield by using the estimated spectral data (q1 through q59), as described below.

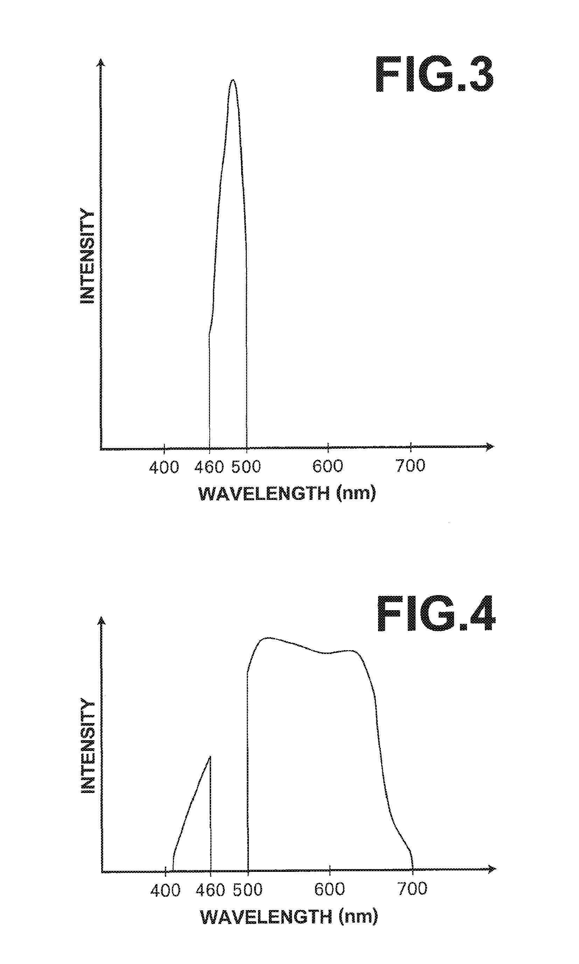

In the image processing unit 182, the following signal processing is performed for each pixel. When the excitation light L2 is output, the pseudo-fluorescence intensity that is the intensity of light in a specific fluorescence wavelength band is calculated based on the estimated spectral data (q11 through q19) of the specific fluorescence wavelength band. The specific fluorescence wavelength band is a wavelength band, such as the wavelength band of 460 nm to 500 nm as illustrated in Figure 3, which includes the wavelength of 480 nm. The wavelength of 480 nm is the center wavelength of fluorescence output from the region 10 to be observed when the region 10 to be observed is illuminated with the excitation light L2. The pseudo-fluorescence intensity does not exactly represent the intensity of the fluorescence. However, as described above, the pseudo-fluorescence intensity includes information about the value of the spectral radiation rate of the fluorescence, i.e., whether the value of the spectral radiation rate is large or small.

As the specific fluorescence wavelength band, a wavelength band that is set in the image processing unit 183 in advance may be used. Alternatively, a wavelength band that is input by an input operation at the input unit 192 may be used.

Further, pseudo-three-color image signals Rs, Gs and Bs are obtained from estimated spectral data of a semi-ordinary wavelength band that does not include the specific fluorescent wavelength band. For example, the semi-ordinary wavelength band is a wavelength band of 410 nm to 460 nm and 500 nm to 700 nm, as illustrated in Figure 4. In this case, for example, the intensity of light is calculated from the estimated spectral data of the wavelength band of 410 nm to 460 nm, and the calculated value is determined as a Bs signal. Further, the intensity of light is calculated from the estimated spectral data of the wavelength band of 500 nm to 600 nm, and the calculated value is determined as a Gs signal. Further, the intensity of light is calculated from the estimated spectral data of the wavelength band of 600 nm to 700 nm, and the calculated value is determined as an Rs signal.

These pseudo-three color image signals Rs, Gs and Bs are used to generate a Y/C signal (pseudo-color ordinary image signal) composed of a luminance signal Y and chrominance signals C. Further, the generated Y/C signal is output as a pseudo-color ordinary image signal to the display processing unit 184.

The intensity (radiation intensity) of the fluorescence output from a fluorescent substance is substantially proportional to the illumination intensity of the excitation light, and the illumination intensity of the excitation light decreases in inverse proportion to the square of the distance. Therefore, in some cases, the intensity of fluorescence received from a lesion tissue that is located close the light source is higher than the intensity of fluorescence received from a normal tissue located far from the light source. Therefore, it is impossible to represent the tissue characteristic of the region to be observed only by the information about the intensity of the received fluorescence. Therefore, conventionally, light in a wavelength band that is different from the wavelength band of the excitation light is output to the region to be observed as reference light, and the intensity of light reflected from the region to be observed (hereinafter, referred to as reference light intensity) that has been illuminated with the reference light is detected. Further, the intensity of fluorescence is divided by the intensity of the reference light to obtain a fluorescence yield. Further, the fluorescence image is produced based on the fluorescence yield.

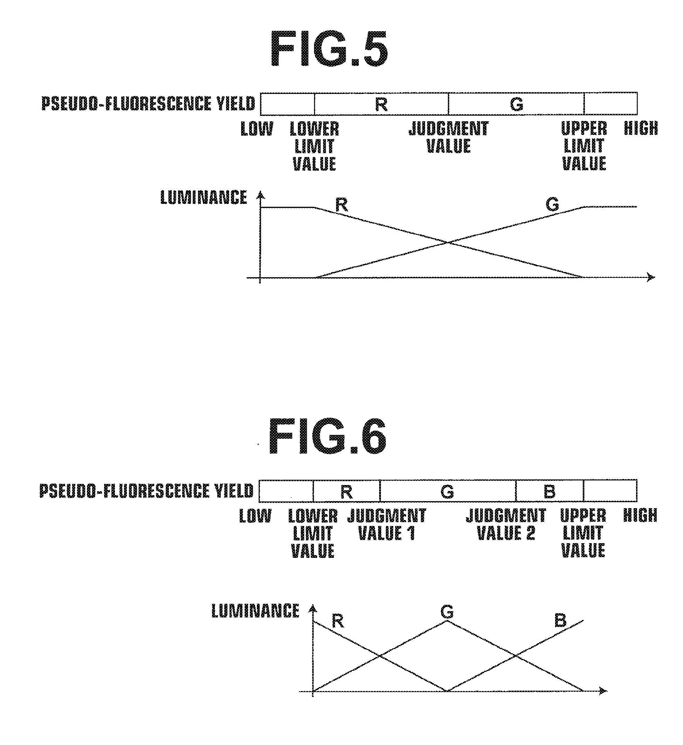

In the image processing unit 182, a pseudo-fluorescence yield is obtained by using the value of the luminance signal Y of the pseudo-color ordinary image signal, as the aforementioned reference light intensity. Specifically, the pseudo-fluorescence yield is obtained by dividing the pseudo-fluorescence intensity by the value of the illumination signal Y of the pseudo-color ordinary image signal. Further, a fluorescence image is produced by assigning green, or red or the like to the pseudo-fluorescence yield. For example, as illustrated in Figure 5, if the pseudo-fluorescence yield is greater than or equal to a predetermined judgment value, green is assigned. If the pseudo-fluorescence yield is lower than the predetermined judgment value, red is assigned to produce the fluorescent image. Alternatively, red and green may be mixed by using an additive color mixing method, and a fluorescent image, the display color of which sequentially changes in the order of red, yellow and green based on the value of the pseudo-fluorescence yield, may be produced. Further, when the value of the pseudo-fluorescence yield is less than or equal to a predetermined lower limit value, only red is assigned. When the value of the pseudo-fluorescence yield is higher than or equal to a predetermined upper limit value, green is assigned. Accordingly, the lesion tissue that has a smaller pseudo-fluorescence yield is display in red, and a normal tissue that has a larger pseudo-fluorescence yield is displayed in green.

Alternatively, as illustrated in Figure 6, a fluorescent image may be produced by assigning red, green or blue to the pseudo-fluorescence yield by comparing the pseudo-fluorescence yield with a judgment value or values. Further, red, green and blue may be mixed by using an additive color mixing method, and a fluorescent image, the display color of which sequentially changes in the order of red, yellow, green, cyan and blue based on the value of the pseudo-fluorescence yield may be produced. When the pseudo-fluorescence yield is less than or equal to a predetermined lower limit value or higher than or equal to a predetermined upper limit value, an achromatic color may be assigned.

In the present embodiment, the value of the luminance signal Y of the pseudo-color ordinary image signal was used as the intensity of reference light. However, instead of the value of the luminance signal Y of the pseudo-color ordinary image signal, the intensity of light of the image signal Rs, the intensity of light obtained from estimated spectral data in a long wavelength band (for example at 620 nm or the like) may be used. In the long wavelength band, a difference between the intensity of fluorescence output from the normal tissue and the intensity of fluorescence output from the lesion tissue is small.

The image processing unit 182 generates fluorescence superimposed image data in which the aforementioned fluorescent image is superimposed on an image that reflects only the luminance signal Y of the pseudo-color ordinary image signal, in other words, a pseudo-black-and-white ordinary image so that the user who observes the image can easily identify or confirm the position of the lesion tissue, which has a small pseudo-fluorescence yield. The image processing unit 182 outputs the fluorescence superimposed image data to the display processing unit 184. In the display processing unit 184, a display image that displays the pseudo-color ordinary image data and the fluorescence superimposed image data output from the image processing unit 182 next to each other is produced, or a color image signal for display in which the pseudo-color ordinary image data and the fluorescence superimposed image data are combined (synthesized) as a single image is produced, and output to the monitor 11 to be displayed.

Further, the processor unit 172 may judge in advance whether the pseudo-fluorescence yield at each of all pixels is greater than or equal to a predetermined judgment value. When the pseudo-fluorescence yield is greater than or equal to the predetermined value, in other words, when a portion corresponding to lesion tissue is not present in the image, only the pseudo-color ordinary image data may be displayed.

Further, the image processing unit 182 outputs the luminance signal Y of the pseudo-color ordinary image signal for each pixel or an average luminance signal Y' of a plurality of adjacent pixels to the light amount control unit 186. The light amount control unit 186 calculates an average luminance value Ya of pixels in a specified area for each frame, and compares the average luminance value Ya with a reference luminance value Yr that has been stored in the memory 190 in advance. Further, the light amount control unit 186 selects a diaphragm control signal based on the result of comparison, and outputs the diaphragm control signal to the control unit 167 of the illumination light unit 150. At the same time, in the excitation light unit 130, a drive current control signal for controlling the value of drive current supplied from the drive circuit 132 to the semiconductor laser 131 is obtained, and the drive current control signal is output to the control unit 140 of the excitation light unit 130.

As the diaphragm control signal, when the average luminance value Ya is greater than the reference luminance value Yr, a signal that reduces the aperture amount of the diaphragm 156 is selected, in other words, a signal that reduces the light passing through the aperture of the diaphragm 156 is selected. When the average luminance value Ya is smaller than the reference luminance value Yr, a signal that increases the aperture amount of the diaphragm 156 is selected, in other words, a signal that increases the light passing through the aperture of the diaphragm 156 is selected. When the average luminance value Ya and the reference luminance value Yr are substantially the same, a signal that maintains the same aperture amount is selected. Further, a drive current control signal corresponding to the diaphragm control signal is output so that the ratio of the light amount of the illumination light L1 to the light amount of the excitation light L2 becomes a predetermined ratio. The ratio of the light amount of the illumination light L1 to the light amount of the excitation light L2 may be set in advance by an input operation at the input unit 192. The light amount control unit 186 determines the drive current amount of the excitation light L2 based on the set ratio and the aperture amount of the illumination light L1, and outputs the drive current control signal.

Further, the control unit 167 of the illumination light unit 150 controls the aperture amount of the diaphragm 156 based on the diaphragm control signal. Further, the control unit 140 of the excitation light unit 130 controls, based on the drive current control signal, the current value supplied from the drive circuit 132 to the semiconductor laser 131.

As described above, in the fluorescence endoscope apparatus 100 of the present embodiment, the light amount control unit 186 controls the light amount of the illumination light L1 so that the representative luminance value of the ordinary image becomes a predetermined luminance value. Further, the light amount control unit 186 controls the light amount of the excitation light L2 so that the ratio of the light amount of the excitation light L2 to the light amount of the illumination light L1 becomes a predetermined ratio. Therefore, it is possible to prevent the risk of increasing the light amount of the excitation light L2 when the distance between the output end of the excitation light L2 and the region 10 to be observed is short, in other words, when the light amount of the excitation light should not be increased. Therefore, it is possible to obtain a fluorescent image that reflects the tissue characteristic of the region 10 to be observed.

Hence, it is possible to improve the reliability of the fluorescent image.

Further, in the fluorescence endoscope apparatus 100 of the present invention, it is possible to make the number of frames of the pseudo-ordinary color image and the fluorescence superimposed image per unit time the same as the number of frames of the ordinary color image. Therefore, it is possible to produce an efficient display image even when the image is displayed as a motion image.

Further, in the fluorescence endoscope apparatus 100 of the present invention, when the connection of the excitation light unit 130 to the illumination light unit 150 is not detected, in other words, when connection signals are not output from both of the connection detection unit 162 and the connection detection unit 166, the switch 134 provided between the drive circuit 132 and the semiconductor laser 131 in the excitation light unit 130 is kept open. Therefore, electric current is not supplied to the semiconductor laser 131. Hence, even if the user of the fluorescence endoscope apparatus is not used to handling of the light source for excitation light, such as laser, it is possible to safely obtain the fluorescent image.

Further, when the connection of the excitation light unit 130 to the illumination light unit 150 is not detected, even if a predetermined key of the input unit 192 or the switch of the scope unit 110 is pressed, the operation state is switched only between the operation stop state and the ordinary image mode, in other words, the operation state is not switched to the fluorescent image mode. Therefore, the risk of error operations can be prevented, and the convenience of the apparatus is further improved.

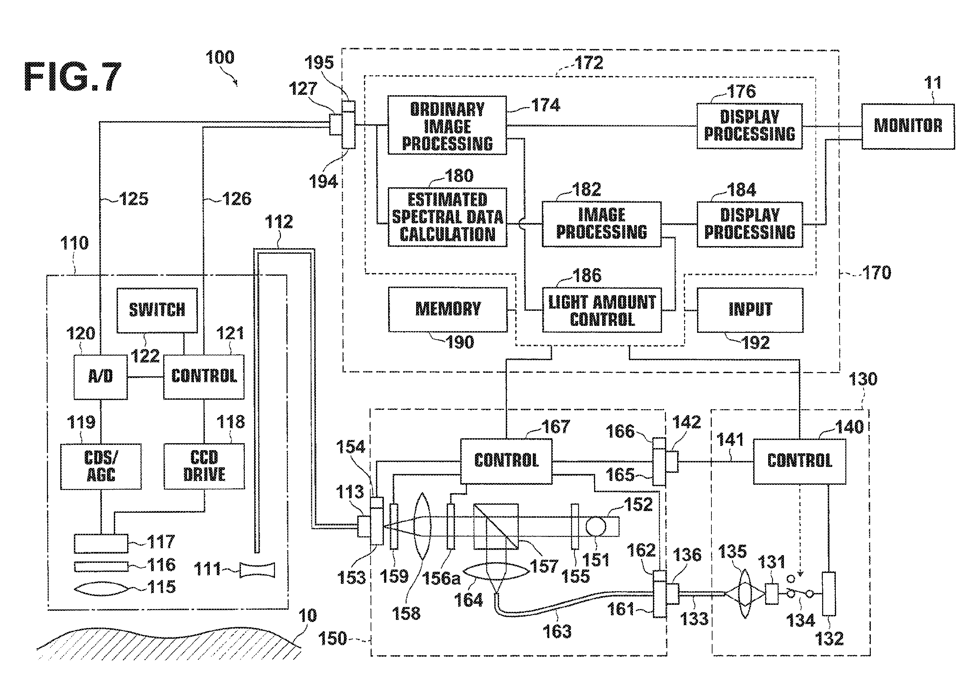

Further, in the present embodiment, the diaphragm 156 is arranged between the xenon lamp 151 and the dichroic mirror 157. However, it is not necessary that the diaphragm 156 is arranged in such a manner. In a modification example, a diaphragm 156a may be arranged between the dichroic mirror 157 and the condensing lens 158, as illustrated in Figure 7. In this case, the light amount of the excitation light L2 is determined by the diaphragm 156a and the value of the drive current supplied from the drive circuit 132 to the semiconductor laser 131. Therefore, the light amount is controlled by taking these factors into consideration in advance.

In the present embodiment, the ordinary image processing unit 174 and the display processing unit 176 that perform signal processing when the ordinary image mode is selected, and the estimated spectral data calculation unit 180, the image processing unit 182 and the display processing unit 184 that perform signal processing when the fluorescent image mode is selected are provided in the processing unit 172. However, the configuration of the processing unit 172 is not limited to the configuration of the present embodiment. For example, an image processing unit that functions as the estimated spectral data calculation unit 180, the ordinary image processing unit 174 and the image processing unit 182 and a display processing unit that functions as the display processing unit 176 and the display processing unit 184 maybe provided. Further, when the ordinary image mode is selected, the signal output from the scope 110 unit may be directly input to the image processing unit, and when the fluorescent image mode is selected, the signal output from the scope unit 110 may be input to the estimated spectral data calculation unit 180.

Further, in the present embodiment, a wavelength band that has a predetermined width and that includes the wavelength of 480 nm, which is the center wavelength band of fluorescence, is used as the specific fluorescence wavelength band. However, the specific fluorescence wavelength band is not limited to this wavelength band. The specific fluorescence wavelength band should substantially reflect the intensity of the fluorescence, and the specific fluorescence wavelength band may be, for example, only the wavelength of 480 nm, only the wavelength of 470 nm, or only the wavelength of 490 nm or the like. Alternatively, the specific fluorescence wavelength band may be the wavelength band of 475 nm to 485 nm or the like. For example, when the specific fluorescence wavelength band is only the wavelength of 480 nm, it is possible to obtain the pseudo-fluorescence intensity by calculating only the estimated spectral data (q15). When the specific fluorescence wavelength band is the wavelength band of 475 nm to 485 nm, it is possible to obtain the pseudo-fluorescence intensity by calculating the estimated spectral data (q14, q15 and q16).

Further, it is desirable that the whole wavelength band of the specific fluorescence wavelength band is within the substantial wavelength band of fluorescence, and it is not desirable that the specific fluorescence wavelength band is unnecessarily wide. Specifically, it is desirable that the width of the specific fluorescence wavelength band is less than or equal to 100 nm, and optionally less than or equal to 50 nm. Further, the width may be less than or equal to 10 nm or a single wavelength as described above.

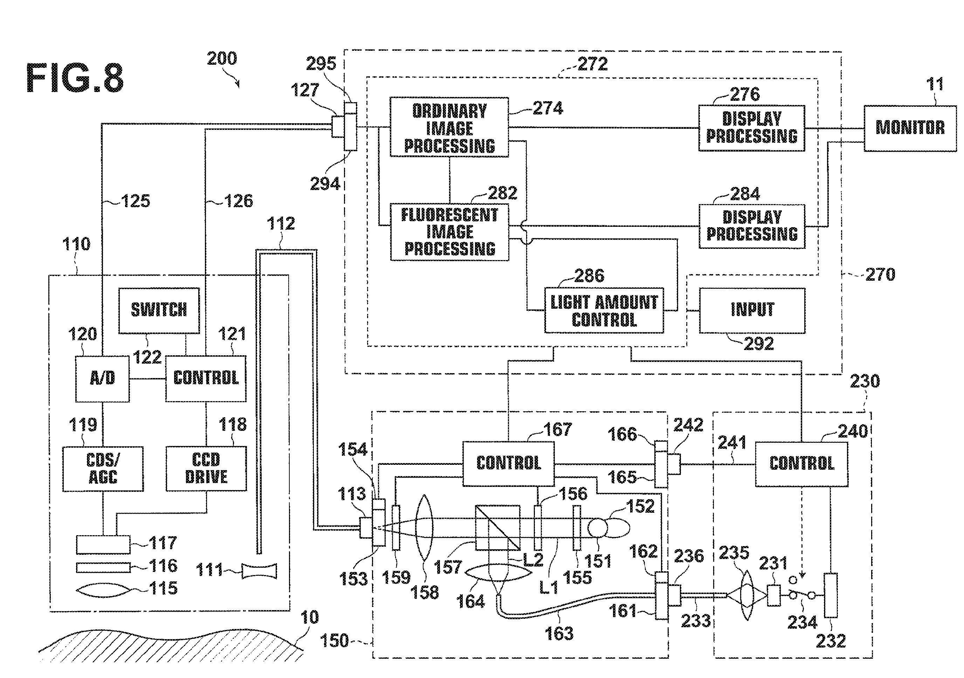

Next, a fluorescence endoscope using a second embodiment of the fluorescent image obtainment apparatus of the present invention will be described in detail. Figure 8 is a schematic diagram illustrating the configuration of the fluorescence endoscope apparatus to which the second embodiment of the present invention has been applied. A fluorescence endoscope apparatus 200 in the present embodiment is configured in such a manner that the operation mode can be switched between an ordinary image mode and a fluorescent image mode. In the ordinary image mode, the region 10 to be observed is illuminated with illumination light L1 to obtain color ordinary images, and the obtained images are displayed as a motion image. In the fluorescent image mode, the region 10 to be observed is illuminated with the illumination light L1 and excitation light L2 to obtain combined images, and fluorescent images are obtained from the combined images by performing operation processing, which will be described later. Further, the obtained fluorescent images and the color ordinary images are superimposed one on the other to obtain fluorescence superimposed images, and the obtained fluorescence superimposed images are displayed as a motion image. In the following description, elements of the fluorescence endoscope apparatus 200 of the second embodiment that are different from the elements of the fluorescence endoscope apparatus 100 of the first embodiment will be mainly described. Further, the same reference numerals are assigned to elements similar to the elements of the fluorescence endoscope apparatus 100 of the first embodiment.

As illustrated in Figure 8, the fluorescence endoscope apparatus 200 includes the scope unit 110, a processor unit 270, the illumination light unit 150, and an excitation light unit 230. The scope unit 110 is inserted into the body cavity of a person to be examined (a patient) to observe the region 10 to be observed. The processor unit 270 is electrically connected to the scope unit 110 in a detachable manner. The illumination light unit 150 is optically connected to the scope unit 110 in a detachable manner, and stores the xenon lamp 151 that outputs the illumination light L1. The excitation light unit 230 is both electrically and optically connected to the illumination light unit 150 in a detachable manner, and stores a GaN-based semiconductor laser 231 that outputs the excitation light L2. The processor unit 170 and the illumination light unit 150 may be structured as a single body or as separate bodies.

The scope unit 110 and the illumination light unit 150 are structured in a manner similar to those of the fluorescence endoscope apparatus of the first embodiment.

The excitation light unit 230 includes a GaN-based semiconductor laser 231 that outputs the excitation light L2, a drive circuit 232 that drives the semiconductor laser 231, and a light guide 233 that guides the excitation light L2 output from the semiconductor laser 231. The light guide 233 extends from the case of the excitation light unit 230, and the other end of the light guide 233 is connected to the optical connector 236. The optical connector 236 is detachably connected to the optical connector 161 of the illumination light unit 150. A switch 234 is provided between the semiconductor laser 231 and the drive circuit 232, and a light-condensing optical system 235 is provided between the semiconductor laser 231 and an end (light incident end) of the light guide 233.

In the fluorescence endoscope apparatus 200 of the present embodiment, the drive circuit 232 that drives the semiconductor laser 231 generates a drive pulse signal, and the semiconductor laser 231 is driven based on the drive pulse signal. The semiconductor laser 231 is driven based on the drive pulse signal, and outputs pulsed excitation light L2. Further, the drive circuit 232 generates a drive pulse signal or a plurality of drive pulse signals in a predetermined time period in which light can be output. The drive circuit 232 generates the drive pulse signal or signals at a predetermined cycle. The semiconductor laser 231 outputs pulsed excitation light based on the drive pulse signal. The drive circuit 232 changes the number of the drive pulse signal or signals in the predetermined time period in which light can be output, or the width (light output time) of the drive pulse signal based on the drive pulse control signal output from the light amount control unit 286, which will be described later. The drive control of the semiconductor laser 231 by the drive circuit 232 will be described later in detail.

Further, the excitation light unit 230 includes a control unit 240 that is connected to each element, such as the drive circuit 232 and the switch 234, provided in the excitation light unit 230, to control each of the elements. Further, the control unit 240 controls communication between the illumination light unit 150 and the excitation light unit 230. The control unit 240 is connected to an end of a signal line 241. The signal line 241 extends to the outside of the case of the excitation light unit 230, and a connector 242 is provided at the other end of the signal line 241. The connector 242 is detachably connected to the connector 165 of the illumination light unit 150.

Meanwhile, a processing unit 272 is provided in the processor unit 270. The processing unit 272 includes an ordinary image processing unit 274 and a display processing unit 276 that perform signal processing when an ordinary image mode is selected. Further, the processing unit 272 includes a fluorescent image processing unit 282 and a display processing unit 284 that perform image processing when a fluorescent image mode is selected. Further, the processing unit 272 includes a light amount control unit 286 that controls the intensity of the illumination light and the excitation light.

The ordinary image processing unit 274 generates an ordinary image signal composed of three-color image signals of R, G and B based on the ordinary image obtained by the scope unit 110 by illumination of the region 10 to be observed with the illumination light L1. Further, the ordinary image processing unit 274 performs various kinds of image processing on the ordinary image signal, and generates a Y/C signal composed of a luminance (Y) signal and chrominance [C (R - Y and B - Y)] signals. Further, the ordinary image processing unit 274 outputs the Y/C signal to the display processing unit 276. The display processing unit 276 performs various kinds of signal processing on the Y/C signal to generate color ordinary image signal for display. The color ordinary image signal is output to a monitor 11, such as a liquid crystal display and a CRT, for example.

When the fluorescent image mode is selected, the fluorescent image processing unit 282 generates a combined image signal composed of three-color image signals of R, G and B based on a combined image obtained by the scope unit 110 in the time period in which the region 10 to be observed is illuminated with both of the illumination light L1 and the excitation light L2. Further, the fluorescent image processing unit 282 subtracts the ordinary image signal obtained by the ordinary image processing unit 274 from the combined image signal to obtain the fluorescent image signal. Further, various kinds of signal processing are performed on the fluorescent image signal, and the ordinary image signal obtained by the ordinary image processing unit 274 and the fluorescent image signal are superimposed one on the other to generate a fluorescence superimposed image signal. Further, after various kinds of signal processing are performed on the fluorescence superimposed image signal, a Y/C signal composed of a luminance (Y) signal and chrominance [C (R - Y, B - Y)] signals is generated and output to the display processing unit 284. The display processing unit 284 performs various kinds of signal processing on the Y/C signal to generate a fluorescence superimposed image signal for display, and outputs the generated fluorescence superimposed image signal for display to the monitor 11.

The light amount control unit 286 is connected to the ordinary image processing unit 274, and controls the light amount of the illumination light L1 and the excitation light L2 based on the luminance signal that is based on the ordinary image signal. The method for controlling the light amount of the illumination light L1 and the excitation light L2 will be described later in detail.

Further, the processing unit 272 is connected to a keyboard-type input unit 292 and a connector 294. The connector 294 is detachably connected to the connector 127 of the scope unit 110. The connector 294 has a connection detection unit 295 that detects whether the connector 127 is connected to the connector 294. Further, the processing unit 272 is connected to the control unit 121 of the scope unit 110, the control unit 167 of the illumination light unit 150, and the control unit 240 of the excitation light unit 230.

Next, the operation of the fluorescence endoscope apparatus according to the present invention will be described. First, an operation in the ordinary image mode will be described. In the ordinary image mode, the region 10 to be observed is illuminated with illumination light L1 to obtain color ordinary images, and the color ordinary images are displayed as a motion image.

Before the fluorescence endoscope apparatus of the present embodiment is used, a washed and sterilized scope unit 110 is attached to the processor unit 270 and the illumination light unit 150. Further, the connector 127 connected to an end of the signal line 125 of the scope unit 110 and an end of the signal line 126 of the scope unit 110 is connected to the connector 294 of the processor unit 270. Further, the optical connector 113 provided at the leading end of the light guide 112 is connected to the optical connector 153 of the illumination light unit 150. When the connector 127 is connected to the connector 294, the connection detection unit 295 provided for the connector 294 sends a connection signal to the processing unit 272. Further, when the optical connector 113 is connected to the optical connector 153, the connection detection unit 154 provided for the optical connector 153 outputs a connection signal to the control unit 167.

When the processing unit 272 receives connection signals from the connection detection unit 295 and the connection detection unit 154, a rotary shutter 159 of the illumination light unit 150 is rotated so that an operation in the ordinary image mode can be performed. Further, the function mode of a predetermined key of the input unit 292 is set through the processing unit 272 of the processor unit 270. Further, the function mode of the switch 122 is set through the processing unit 272 and the control unit 121 of the scope unit 110. Since the processing unit 272 controls processing, the operation mode switches between a stop state and an ordinary image mode when the user presses a predetermined key of the input unit 292 or the switch 122.

When the user presses the predetermined key of the input unit 292 or the switch 122 once, an operation in the ordinary image mode starts. In the illumination light unit 150, the xenon lamp 151 is turned on by the drive circuit 152, and the illumination light L1 is continuously output. The illumination light L1 passes through the wavelength filter 155, the diaphragm 156 and the dichroic mirror 157 and condensed onto the end surface of the optical connector 113 by the condensing lens 158. The condensed light enters the light guide 112. The illumination light L1 propagates through the light guide 112, and is output from the leading end of the light guide 112. The output light continuously illuminates the region 10 to be observed through the optical system 111 for illumination.

The wavelength band of the illumination light L1 is limited to greater than or equal to 410 nm and less than or equal to 700 nm by the wavelength filter 155. The light amount of the illumination light L1 is controlled by the diaphragm 156. The light amount control operation of the illumination light L1 by the diaphragm 156 will be described later.

The CCD 117 that is driven by the CCD drive circuit 118 captures an ordinary image of the region 10 to be observed, and outputs an imaging signal. The imaging signal is amplified in the CDS/AGC circuit 119 by correlated double sampling and automatic gain control. Further, A/D conversion is performed on the amplified imaging signal in the A/D converter 120, and output to the ordinary image processing unit 274 in the processing unit 272 of the processor unit 270. In the ordinary image processing unit 274, when the ordinary image mode is selected, an ordinary image signal composed of three-color image signals of R, G and B is generated based on the signal output from the scope unit 110. Further, the ordinary image processing unit 274 performs various kinds of processing to generate a Y/C signal (color ordinary image signal) that is composed of a luminance signal Y and chrominance signals C. The generated Y/C signal is output to the display processing unit 276. In the display processing unit 276, various kinds of signal processing, such as I/P conversion and noise removal processing, is performed on the Y/C signal, and the processed signal is output to the monitor 11.

The monitor 11 displays the color ordinary images based on the input color ordinary image signal as a motion image.

Further, the ordinary image processing unit 174 outputs the luminance signal Y for each pixel or an average luminance signal Y' of a plurality of adjacent pixels to the light amount control unit 286. The light amount control unit 286 calculates an average luminance value Ya of pixels in a specified area for each frame based on the input luminance signal Y or average luminance signal Y', and compares the average luminance value Ya with a reference luminance value Yr that has been stored in the memory (not illustrated) in advance. Further, the light amount control unit 286 selects a diaphragm control signal based on the result of comparison, and outputs the diaphragm control signal to the control unit 167 of the illumination light unit 150. As the diaphragm control signal, when the average luminance value Ya is greater than the reference luminance value Yr, a signal that reduces the aperture amount of the diaphragm 156 is selected, in other words, a signal that reduces the light passing through the aperture of the diaphragm 156 is selected. When the average luminance value Ya is smaller than the reference luminance value Yr, a signal that increase the aperture amount of the diaphragm 156 is selected, in other words, a signal that increases the light passing through the aperture of the diaphragm 156 is selected. When the average luminance value Ya and the reference luminance value Yr are substantially the same, a signal that maintains the same aperture amount is selected.

The control unit 167 of the illumination light unit 150 controls the aperture amount of the diaphragm 156 based on the diaphragm control signal.

Next, an operation in the fluorescent image mode will be described. Before the fluorescence endoscope apparatus is used in the fluorescent image mode, a washed and sterilized scope unit 110 is attached to the processor unit 270 and the illumination light unit 150. Further, the connector 127 connected to an end of the signal line 125 of the scope unit 110 and an end of the signal line 126 of the scope unit 110 is connected to the connector 294 of the processor unit 270. When the connector 294 is connected to the connector 127, the connection detection unit 295 provided for the connector 294 outputs a connection signal to the processing unit 272. Further, the optical connector 113 provided at the leading end of the light guide 112 is connected to the optical connector 153 of the illumination light unit 150. When the optical connector 153 is connected to the connector 113, the connection detection unit 154 provided for the optical connector 153 sends a connection signal to the control unit 167.

Further, the excitation light unit 230 is connected to the illumination light unit 150. The connector 242 provided at the leading end of the signal line 241 of the excitation light unit 230 is connected to the connector 165 of the illumination light unit 150. When the connector 142 is connected to the connector 165, the connection detection unit 166 provided for the connector 165 outputs a connection signal to the control unit 167. Further, the optical connector 236 provided at the leading end of the light guide 233 is connected to the optical connector 161 of the illumination light unit 150. When the optical connector 236 is connected to the optical connector 161, the connection detection unit 162 provided for the optical connector 161 outputs a connection signal to the control unit 167.

The control unit 240 of the excitation light unit 230 communicates with the control unit 167 of the illumination light unit 150. When the control unit 240 receives connection signals from the connection detection unit 166 and the connection detection unit 162, the control unit 240 closes the switch 234 of the excitation light unit 230 to electrically connect the semiconductor laser 231 and the drive circuit 232. Consequently, the semiconductor laser 231 becomes drivable by the drive circuit 232. Further, the function mode of a predetermined key of the input unit 292 is set through the processing unit 272 of the processor unit 270. Further, the function mode of the switch 122 is set through the processing unit 272 and the control unit 121 of the scope unit 110. Since the processing unit 272 controls processing, the operation mode switches between a stop state, an ordinary image mode and a fluorescent image mode when the user presses a predetermined key of the input unit 292 or the switch 122. When the connection signals are not received from both of the connection detection unit 166 and the connection detection unit 162, in other words, when no connection signals are received from the two connection detection units, or no connection signal is received from one of the connection detection units, the switch 234 in the excitation light unit 230 is constantly open. Hence, when the excitation light unit 230 is not connected to the illumination light unit 150, the semiconductor laser 231 is not driven.

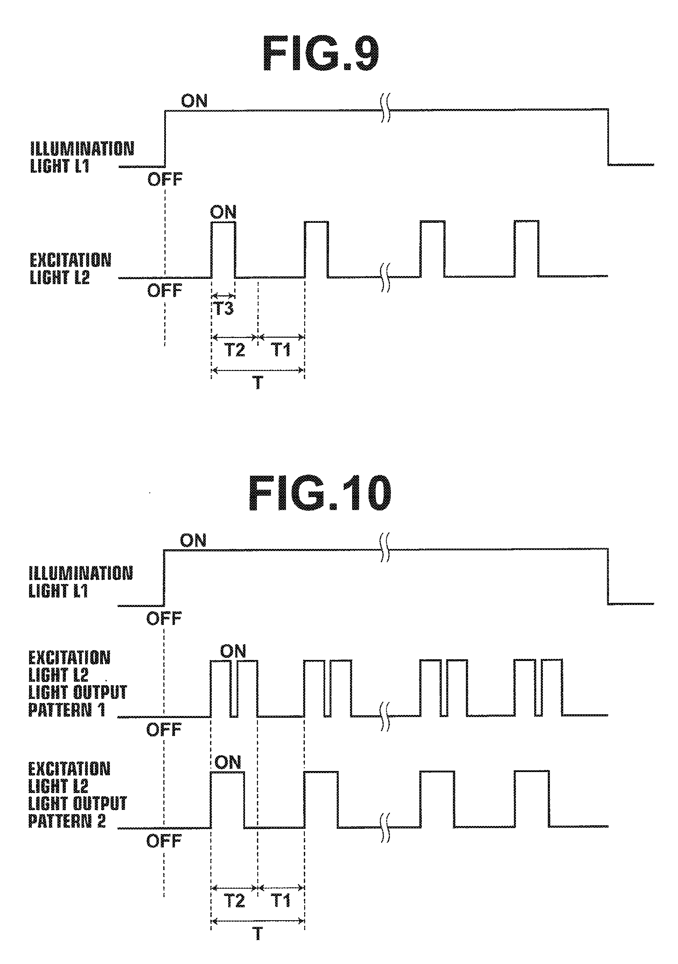

Further, when the fluorescence endoscope apparatus is operating in the ordinary image mode, if the user presses the predetermined key of the input unit 292 or the switch 122 once, the operation in the fluorescent image mode starts. In addition to the operation of the illumination light unit 150, the operation of the excitation light unit 230 starts. The semiconductor laser 231 is pulse-driven based on the drive pulse signal output from the drive circuit 232, and excitation light L2 that has a wavelength of 405 nm is output. Specifically, as illustrated in Figure 9, pulsed excitation light that has pulse width T3 is output, at cycle T, in time period T2 in which light can be output.

The excitation light L2 is condensed by the condensing lens 235, and enters the end surface of the light guide 233. The excitation light L2 propagates through the light guide 233, and enters the light guide 163 through the optical connector 236 and the optical connector 161.

The excitation light L2 propagates through the light guide 163, and is output from an end of the light guide 163. The output excitation light L2 is collimated by the collimator lens 164, and enters the dichroic mirror 157. Since the wavelength of the excitation light L2 is 405 nm, the excitation light L2 is reflected at a right angle from the dichroic mirror 157, and condensed onto the end surface of the optical connector 113 by the condensing lens 158. The condensed light enters the light guide 112.

The excitation light L2 propagates through the light guide 112, and is output from the leading end of the light guide 112 . Further, the excitation light L2 is output to the region 10 to be observed through the optical system 111 for illumination to illuminate the region 10 to be observed. In this case, the region 10 to be observed is illuminated with both of the illumination light L1 and the excitation light L2 at the same time.

The CCD 117 that is driven by the CCD drive circuit 118 captures an ordinary image composed of the reflection light of the illumination light L1, reflected from the region 10 to be observed, in time period T1 illustrated in Figure 9. In the time period T1, only the illumination light L1 is output. Further, in time period T2 illustrated in Figure 9, in which light can be output, the CCD 117 captures a combined image composed of the reflection light of the illumination light L1, reflected from the region 10 to be observed, and fluorescence output from the region 10 to be observed by illumination with the excitation light L2. Further, since an excitation light cut filter that cuts light that has a wavelength lower than or equal to 410 nm is provided at the leading end of the CCD 117, most of the reflection light of the excitation light L2 does not enter the CCD 117.

The CCD 117 alternately outputs the imaging signal obtained in the time period T1 and the imaging signals obtained in the time period T2, in which light can be output. Further, these imaging signals are amplified in the CDS/AGC circuit 119 by correlated double sampling and automatic gain control. Further, A/D conversion is performed on the amplified imaging signals in the A/D converter 120, and output as an RGB image signal to the processing unit 272 of the processor unit 270. At this time, the RGB image signal based on the ordinary image obtained in the time period T1 is input to the ordinary image processing unit 274, and the RGB image signal based on the combined image obtained in the time period T2, in which light can be output, is input to the fluorescent image processing unit 282.

Further, the ordinary image processing unit 274 generates an ordinary image signal from the input RGB image signal, and outputs the ordinary image signal to the fluorescent image processing unit 282.

Meanwhile, the fluorescent image processing unit 282 generates a combined image signal from the input RGB image signal. Further, the fluorescent image processing unit 282 subtracts the ordinary image signal from the combined image signal to generate a fluorescent image signal. Further, the fluorescent image processing unit 282 performs various kinds of signal processing on the fluorescent image signal, and superimposes the ordinary image signal on the fluorescent image signal to generate a fluorescence superimposed image signal. Further, the fluorescent image processing unit 282 performs various kinds of signal processing on the fluorescence superimposed image signal, and generates a Y/C signal composed of a luminance (Y) signal and chrominance [C (R - Y, B - Y)] signals. The generated Y/C signal is output to the display processing unit 284 . The display processing unit 284 performs various kinds of signal processing on the Y/C signal to generate a fluorescence superimposed image signal for display, and outputs the generated fluorescence superimposed image signal for display to the monitor 11.

Further, the monitor 11 displays, as a motion image, the fluorescence superimposed image in which the fluorescent image and the ordinary image are superimposed one on the other. The motion image is displayed based on the fluorescence superimposed image signal for display that has been input.

Further, the ordinary image processing unit 274 outputs the luminance signal Y for each pixel or an average luminance signal Y' of a plurality of adjacent pixels to the light amount control unit 286. The light amount control unit 286 calculates an average luminance value Ya of pixels in a specified area for each frame, and compares the average luminance value Ya with a reference luminance value Yr that has been stored in the memory (not illustrated) in advance. Further, the light amount control unit 286 selects a diaphragm control signal based on the result of comparison, and outputs the diaphragm control signal to the control unit 167 of the illumination light unit 150. As the diaphragm control signal, when the average luminance value Ya is greater than the reference luminance value Yr, a signal that reduces the aperture amount of the diaphragm 156 is selected, in other words, a signal that reduces the light passing through the aperture of the diaphragm 156 is selected. When the average luminance value Ya is smaller than the reference luminance value Yr, a signal that increase the aperture amount of the diaphragm 156 is selected, in other words, a signal that increases the light passing through the aperture of the diaphragm 156 is selected. When the average luminance value Ya and the reference luminance value Yr are substantially the same, a signal that maintains the same aperture amount is selected.

Further, the light amount control unit 286 outputs a drive pulse control signal that corresponds to the diaphragm control signal to the drive circuit 232 so that the ratio between the light amount of the illumination light L1 to the excitation light L2 becomes a predetermined ratio. Further, the drive circuit 232 changes the pulse width (the length of light output time) of the drive pulse signal based on the input drive pulse control signal. Alternatively, the drive circuit 232 changes the number of drive pulse signal or signals generated in the predetermined time period T2 in which light can be output. Further, the semiconductor laser 231 outputs pulsed excitation light based on the drive pulse signal. Specifically, the light amount of the excitation light L2 in the time period T2 in which light can be output is changed by changing the number of beams (or the number of times of output) of the pulsed excitation light in the time period T2 in which light can be output as in the light output pattern 1 of the excitation light L2, illustrated in Figure 10. Alternatively, the light amount of the excitation light L2 in the time period T2 in which light can be output is changed by changing the pulse width (pulsed excitation light output time period) of the pulsed excitation light as in the light output pattern 2 of the excitation light L2, illustrated in Figure 10. At this time, when the control signal with respect to the illumination light L1 sent to the diaphragm 156 is a signal to reduce the light amount, the pulse width of the pulsed excitation light is reduced (narrowed) or the number of beams (the number of times of output) of the pulsed excitation light in the time period T2 in which light can be output is reduced. Accordingly, it is possible to reduce the light amount of the excitation light L2 in the time period T2 in which light can be output. Further, when the control signal with respect to the illumination light L1 sent to the diaphragm 156 is a signal to increase the light amount, the pulse width of the pulsed excitation light is increased (widened) or the number of beams of the pulsed excitation light in the time period T2 in which light can be output is increased. Accordingly, it is possible to increase the light amount of the excitation light L2 in the time period T2 in which light can be output. The above description started with the light output pattern illustrated in Figure 9. However, the initial setting of the light output pattern of the pulsed excitation light may be the light output pattern 1 or the light output pattern 2 illustrated in Figure 10 or the like. Further, the light amount may be controlled by increasing or reducing the number of beams of the pulsed excitation light or the width of the pulsed excitation light.

Further the ratio of the light amount of the illumination light L1 to the light amount of the excitation light L2 may be set by an input operation at the input unit 292 in advance. The light amount control unit 286 determines the pulse width and the number of beams of the pulsed excitation light in the time period T2 in which light can be output based on the set ratio and the aperture amount of the illumination light L1. Further, a drive pulse control signal is output based on the pulse width or the number of beams of the pulsed excitation light.

The control unit 167 of the illumination light unit 150 controls the aperture amount of the diaphragm 156 based on the diaphragm control signal. Further, the control unit 240 of the excitation light unit 230 controls, based on the drive pulse control signal, the drive pulse signal supplied from the drive circuit 232 to the semiconductor laser 231.

In the above embodiments, the light amount of the excitation light L2 in the time period T2 in which light can be output is controlled by controlling the pulse width of the pulsed excitation light or the number of beams of the pulsed excitation light in the time period T2. Alternatively, the light amount of the excitation light L2 may be controlled by controlling both of the pulse width of the pulsed excitation light and the number of beams of the pulsed excitation light in the time period T2 at the same time.

Further, in the above description, the fluorescent image signal and the ordinary image signal were superimposed one on the other to generate a fluorescence superimposed image signal. Further, the fluorescence superimposed image for display was displayed based on the fluorescence superimposed image signal. However, it is not necessary that the processing is performed in such a manner. For example, a fluorescence yield may be obtained by dividing the fluorescent image signal by the value of the luminance signal Y of the ordinary image signal. Further, the colors of red, yellow and green may be sequentially assigned to the fluorescence yield based on the size (value) to produce a fluorescent image for display. Further, the produced fluorescent image for display may be displayed on the monitor 11.

Further, the characteristic that the intensity of fluorescence output from normal tissue and the intensity of fluorescence output from lesion tissue differ from each may be utilized, and a judgment unit that judges whether the region 10 to be observed is a lesion tissue based on the fluorescence yield that is obtained as described above may be provided. When the judgment unit judges that the region 10 to be observed is a normal tissue, an ordinary image may be displayed. When the judgment unit judges that the region 10 to be observed is a lesion tissue, a new fluorescent image may be produced by changing the ordinary image color of the pixel based on the fluorescence yield, and displayed.

Further, with respect to the ordinary image, a new ordinary image may be produced by applying a predetermined gain and/or offset to the ordinary image, and the produced image may be displayed. In this case, it is desirable that the gain and/or offset for the ordinary image differs from the gain and/or offset for the fluorescent image. For example, the gains and the offsets may satisfy the following relations: gain GAINS for the fluorescent image > gain GAINn for the ordinary image; and offset OFFSETf for the fluorescent image > offset OFFSETn for the ordinary image.

Further, when the fluorescent image for display is produced, the fluorescent image signal maybe added to the combined image signal to emphasize the fluorescence.

Further, in the fluorescence endoscope apparatus of the above embodiments, the ordinary image signal is generated based on the ordinary image obtained in the time period when only the illumination light L1 is output, and the fluorescent image signal is generated based on the combined image obtained in the period when both of the illumination light L1 and the excitation light L2 are output. Since the time period in which the ordinary image is obtained and the time period in which the combined image is obtained differ from each other, the imaging position of the region 10 to be observed corresponding to the ordinary image and the imaging position of the region 10 to be observed corresponding to the combined image may differ from each other in some cases. In such cases, the shift (difference) in the imaging positions may be detected based on the ordinary image signal that is based on the ordinary image and the combined image signal that is based on the combined image. Further, positioning may be performed based on the shift amount in position. For example, the positions may be adjusted by performing shift correction processing on one of the image signals.

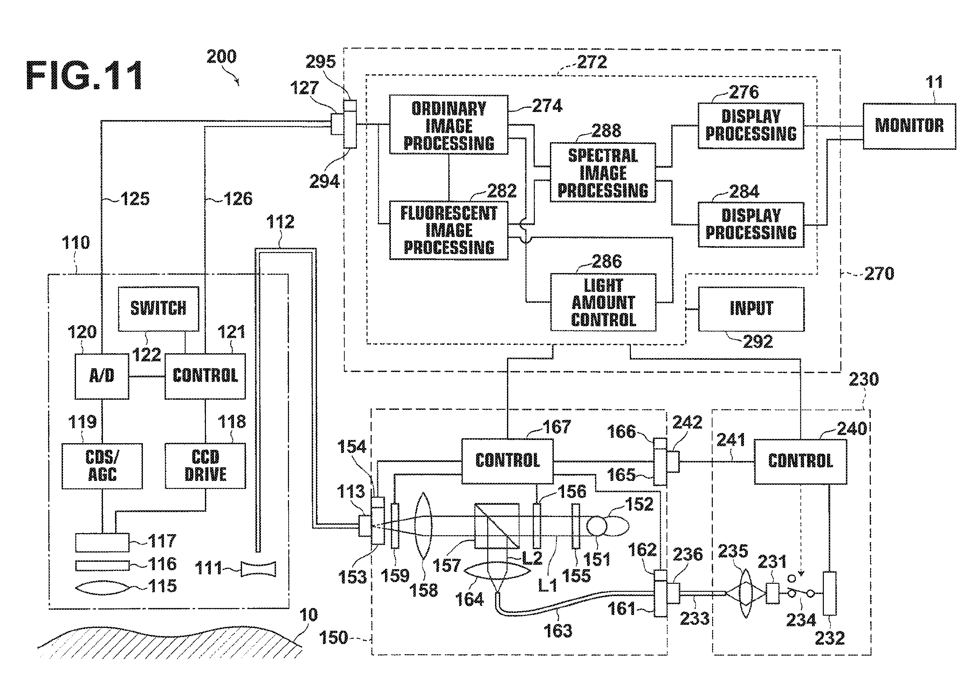

Further, as illustrated in Figure 11, a spectral image processing unit 288 may be provided in the fluorescence endoscope apparatus of the above embodiments. The spectral image processing unit 288 may perform spectral image processing on at least one of the ordinary image signal composed of RGB image signals, obtained by the ordinary image processing unit 274, the combined image signal and the fluorescent image signal that are composed of RGB image signals, and which are obtained by the fluorescent image processing unit 282. Further, whether the spectral image processing is performed may be selected by an input operation at the input unit 292.

Next, the action in the case of performing spectral image processing on the ordinary image signal, the fluorescent image signal or the combined image signal will be described.

The spectral image processing unit 288 performs matrix operation represented by the aforementioned formula 1 for each pixel to generate estimated spectral data (q1 through q59) with respect to the three-color image signal of R, G and B of the ordinary image signal, the fluorescent image signal or the combined image signal. The estimated spectral data is generated by using a matrix of $3 \times 59$ including all of parameters of estimated matrix data stored in the memory (not illustrated).

Here, the estimated matrix data is stored as a table in the memory (not illustrated) in advance, as described above. The example of the estimated matrix data stored in the memory in the present embodiment is shown in the aforementioned Table 1.

For example, three wavelength bands of $\lambda$1, $\lambda$2, $\lambda$3 are selected by an operation at the input unit 292, and parameters corresponding to the selected three wavelength bands are read out from the estimation matrix data stored in the memory.

For example, when the wavelengths of 500 nm, 620 nm and 650 nm are selected as the three wavelength bands $\lambda$1, $\lambda$2, and $\lambda$3, the coefficients of the parameters p21, p45 and p51 in Table 1 that correspond to the wavelengths are used, and matrix operation of the above formula 1 is performed on the RGB signals of the ordinary image signal, the fluorescent image signal or the combined image signal. Accordingly, spectral estimated data $\lambda$1s, $\lambda$2s, $\lambda$3s is calculated.

Further, an appropriate gain and/or offset is applied to each of the calculated spectral estimated data $\lambda$1s, $\lambda$2s, $\lambda$3s, and pseudo-color spectral estimated data $\lambda$1t, $\lambda$2t, $\lambda$3t is calculated. Each of the pseudo-color spectral estimated data $\lambda$1t, $\lambda$2t, $\lambda$3t is used as image signals R', G' and B', respectively.

Further, the pseudo-three-color image signals R', G' and B' are used, and a Y/C signal composed of a luminance signal Y and chrominance signals C is generated, and output to the display processing unit 284. The display processing unit 284 performs various kinds of signal processing on the Y/C signals to generate pseudo-color image signal. The pseudo-color image signal is output to the monitor 11.

Further, the spectral image processing unit 288 may obtain a fluorescence yield, and produce a fluorescent image based on the fluorescence yield.

Specifically, the spectral image processing unit 288 uses, as reference light intensity, the value of the luminance signal Y obtained by the spectral image processing with respect to the ordinary image signal. Further, the spectral image processing unit 288 divides the luminance signal Y' obtained by spectral image processing on the fluorescent image by the value of the luminance signals to obtain an estimated fluorescence yield. Further, a fluorescent image for display is produced by sequentially assigning red, yellow or green to the estimated fluorescence yield, for example, as illustrated in Figure 5. In this case, the lesion tissue that has a smaller estimated fluorescence yield is display in red, and a normal tissue that has a larger estimated fluorescence yield is displayed in green. When the estimated fluorescence yield is lower than or equal to a predetermined lower limit value, only red is assigned. When the estimated fluorescence yield is greater than or equal to a predetermined upper limit value, green is assigned. Alternatively, as illustrated in Figure 6, red, yellow, green, cyan or blue maybe assigned to the estimated fluorescence yield. Further, when the estimated fluorescence yield is less than or equal to a predetermined lower limit value or higher than or equal to a predetermined upper limit, an achromatic color may be assigned.

In the present embodiment, the value of the luminance signal Y obtained by performing spectral image processing on the ordinary image signal was used as the intensity of reference light. However, instead of the value of the luminance signal Y, the intensity of light obtained from estimated spectral data in a long wavelength band, for example at 620 nm or the like, may be used. In the long wavelength band, a difference between the intensity of fluorescence output from the normal tissue and the intensity of fluorescence output from the lesion tissue is small.

For example, the spectral image processing unit 288 generates fluorescence superimposed image for display so that the user who observes the image can easily identify or confirm the position of the lesion tissue, which has a small estimated fluorescence yield. The fluorescence superimposed image for display is produced by assigning data that has been obtained by applying an arbitrary gain or offset to the estimated fluorescence yield to one or two of R, G, and B to obtain a fluorescent image and by superimposing the fluorescent image on a black-and-white ordinary image that reflects the luminance signal Y of the ordinary image. The obtained fluorescence superimposed image for display is output to the display processing unit 284. The display processing unit 284 may produce a display image in which the color ordinary image and the fluorescence superimposed image for display that have been output from the spectral image processing unit 288 are displayed next to each other, and output the display image to the monitor 11 to display the image.

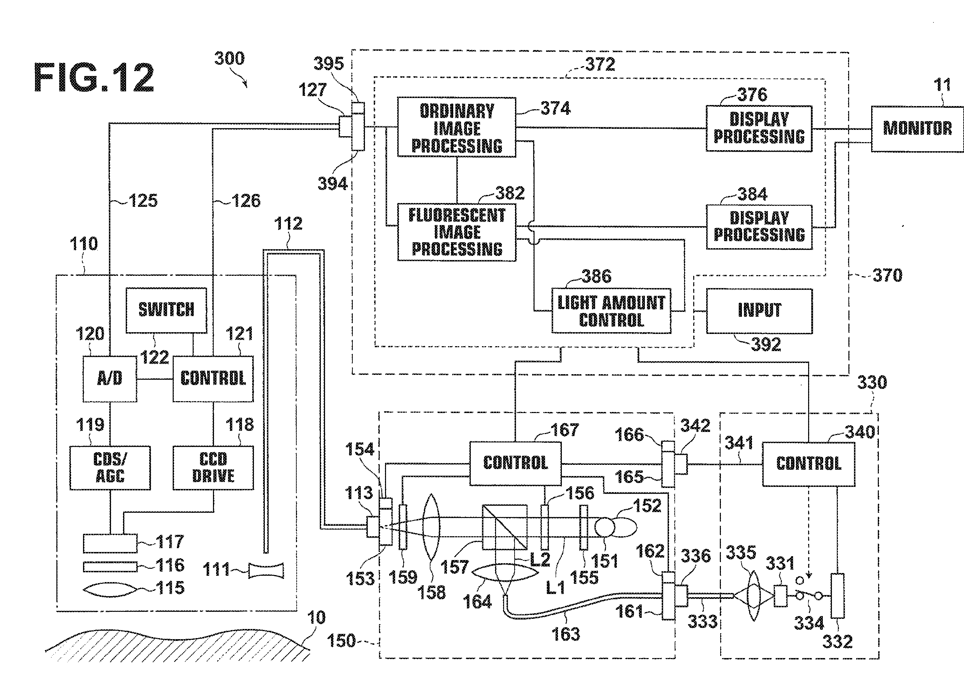

Next, a fluorescence endoscope apparatus using a third embodiment of the fluorescent image obtainment apparatus of the present invention will be described in detail. Figure 12 is a schematic diagram illustrating the configuration of the fluorescence endoscope apparatus to which the third embodiment of the present invention has been applied. A fluorescence endoscope apparatus 300 of the present embodiment is configured in such a manner that the operation mode can be switched between an ordinary image mode and a fluorescent image mode. In the ordinary image mode, the region 10 to be observed is illuminated with illumination light L1 to obtain color ordinary images and the obtained images are displayed as a motion image. In the fluorescent image mode, the region 10 to be observed is illuminated with the illumination light L1 and excitation light L2 to obtain combined images, and fluorescent images are obtained from the combined images by performing operation processing, which will be described later. Further, the obtained fluorescent images and the color ordinary images are superimposed one on the other to obtain fluorescence superimposed images, and the obtained fluorescence superimposed images are displayed as a motion image. In the following description, elements of the fluorescence endoscope apparatus 300 of the present embodiment that are different from the elements of the fluorescence endoscope apparatus of the first embodiment will be mainly described. Further, the same reference numerals are assigned to elements similar to the elements of the fluorescence endoscope apparatus of the first embodiment.

As illustrated in Figure 12, the fluorescence endoscope apparatus 300 includes the scope unit 110, a processor unit 370, the illumination light unit 150, and an excitation light unit 330. The scope unit 110 is inserted into the body cavity of a person to be examined (a patient) to observe the region 10 to be observed. The processor unit 370 is electrically connected to the scope unit 110 in a detachable manner. The illumination light unit 150 is optically connected to the scope unit 110 in a detachable manner, and stores the xenon lamp 151 that outputs the illumination light L1. The excitation light unit 330 is both electrically and optically connected to the illumination light unit 150 in a detachable manner, and stores a GaN-based semiconductor laser 331 that outputs the excitation light L2. The processor unit 370 and the illumination light unit 150 may be structured as a single body or as separate bodies.

The scope unit 110 and the illumination light unit 150 are structured in a manner similar to those of the fluorescence endoscope of the first embodiment.

The excitation light unit 330 includes a GaN-based semiconductor laser 331 that outputs the excitation light L2, a drive circuit 332 that drives the semiconductor laser 331, and a light guide 333 that guides the excitation light L2 output from the semiconductor laser 331. The light guide 333 extends from the case of the excitation light unit 330 to the outside of the excitation light unit 330, and the other end of the light guide 333 is connected to the optical connector 336. The optical connector 336 is detachably connected to the optical connector 161 of the illumination light unit 150. A switch 334 is provided between the semiconductor laser 331 and the drive circuit 332, and a condensing lens 335 is provided between the semiconductor laser 331 and an end (light incident end) of the light guide 333.

In the fluorescence endoscope apparatus 300 of the present embodiment, the drive circuit 332 that drives the semiconductor laser 331 generates a drive pulse signal, and drives the semiconductor laser 331 based on the drive pulse signal. The semiconductor laser 331 is driven based on the drive pulse signal, and outputs pulsed excitation light L2.

Further, the excitation light unit 330 includes a control unit 340 that is connected to each element, such as the drive circuit 332 and the switch 334, provided in the excitation light unit 330, and that controls each of the elements. Further, the control unit 340 controls communication between the illumination light unit 150 and the excitation light unit 330. The control unit 340 is connected to an end of a signal line 341. The signal line 341 extends to the outside of the case of the excitation light unit 330, and a connector 342 is provided at the other end of the signal line 341. The connector 342 is detachably connected to the connector 165 of the illumination light unit 150.

Meanwhile, a processing unit 372 is provided in the processor unit 370. The processing unit 372 includes an ordinary image processing unit 374 and a display processing unit 376 that perform signal processing when an ordinary image mode is selected. Further, the processing unit 372 includes a fluorescent image processing unit 382 and a display processing unit 384 that perform image processing when a fluorescent image mode is selected. Further, the processing unit 372 includes a light amount control unit 386 that controls the intensity of the illumination light and the excitation right.

The ordinary image processing unit 374 generates an ordinary image signal composed of three-color image signals of R, G and B based on an ordinary image obtained by the scope unit 110 by illumination of the region 10 to be observed with the illumination light L1. Further, the ordinary image processing unit 374 performs various kinds of image processing on the ordinary image signal, and generates a Y/C signal composed of a luminance (Y) signal and chrominance [C (R - Y and B - Y)] signals. Further, the ordinary image processing unit, 374 outputs the Y/C signal to the display processing unit 376. The display processing unit 376 performs various kinds of signal processing on the Y/C signal to generate a color ordinary image signal for display. The color ordinary image signal is output to a monitor 11, such as a liquid crystal display or a CRT, for example.

When the fluorescent image mode is selected, the fluorescent image processing unit 382 generates a combined image signal composed of three-color image signals of R, G and B based on a combined image obtained by the scope unit 110 in the time period in which the region 10 to be observed is illuminated with both of the illumination light L1 and the excitation light L2. Further, the fluorescent image processing unit 382 subtracts the ordinary image signal obtained by the ordinary image processing unit 374 from the combined image signal to obtain a fluorescent image signal. Further, various kinds of signal processing are performed on the fluorescent image signal, and the ordinary image signal obtained by the ordinary image processing unit 374 and the fluorescent image signal are superimposed one on the other to generate a fluorescence superimposed image signal. Further, after various kinds of signal processing are performed on the fluorescence superimposed image signal, a Y/C signal composed of a luminance (Y) signal and chrominance [C (R - Y, B - Y)] signals is generated and output to the display processing unit 384. The display processing unit 384 performs various kinds of signal processing on the Y/C signal to generate a fluorescence superimposed image signal for display, and outputs the generated fluorescence superimposed image signal for display to the monitor 11.

The light amount control unit 386 is connected to the ordinary image processing unit 374, and controls the light amount of the illumination light L1 and the excitation light L2 based on the luminance signal that is based on the ordinary image signal.

Further, the processing unit 372 is connected to a keyboard-type input unit 392 and a connector 394. The connector 394 is detachably connected to the connector 127 of the scope unit 110. The connector 394 has a connection detection unit 395 that detects whether the connector 127 is connected to the connector 394. Further, the processing unit 372 is connected to the control unit 121 of the scope unit 110, the control unit 167 of the illumination light unit 150, and the control unit 340 of the excitation light unit 330.

Next, the operation of the fluorescence endoscope apparatus according to the present invention will be described. First, an operation in the ordinary image mode will be described. In the ordinary image mode, the region 10 to be observed is illuminated with the illumination light L1 to obtain color ordinary images, and the color ordinary images are displayed as a motion image.

Before the fluorescence endoscope apparatus of the present embodiment is used, a washed and sterilized scope unit 110 is attached to the processor unit 370 and the illumination light unit 150 . Further, the connector 127 connected to an end of the signal line 125 and an end of the signal line 126 of the scope unit 110 is connected to the connector 394 of the processor unit 370. Further, the optical connector 113 provided at the leading end of the light guide 112 is connected to the optical connector 153 of the illumination light unit 150. When the connector 127 is connected to the connector 394, the connection detection unit 395 provided for the connector 394 sends a connection signal to the processing unit 372. Further, when the optical connector 113 is connected to the optical connector 153, the connection detection unit 154 provided for the optical connector 153 outputs a connection signal to the control unit 167.

When the processing unit 372 receives connection signals from the connection detection unit 395 and the connection detection unit 154, a rotary shutter 159 of the illumination light unit 150 is rotated so that an operation in the ordinary image mode can be performed. Further, the function mode of a predetermined key of the input unit 392 is set through the processing unit 372 of the processor unit 370. Further, the function mode of the switch 122 is set through the processing unit 372 and the control unit 121 of the scope unit 110. Since the processing unit 372 controls processing, the operation mode switches between a stop state and an ordinary image mode when the user presses a predetermined key of the input unit 392 or the switch 122.

When the user presses the predetermined key of the input unit 392 or the switch 122 once, an operation in the ordinary image mode starts. In the illumination light unit 150, the xenon lamp 151 is turned on by the drive circuit 152, and the illumination light L1 is continuously output . The illumination light L1 passes through the wavelength filter 155, the diaphragm 156 and the dichroic mirror 157 and condensed onto the end surface of the optical connector 113 by the condensing lens 158. The condensed light enters the light guide 112. The illumination light L1 that has propagated through the light guide 112 is output from the leading end of the light guide 112. The output light continuously illuminates the region 10 to be observed through the optical system 111 for illumination.

The wavelength band of the illumination light L1 is limited to greater than or equal to 410 nm and less than or equal to 700 nm by the wavelength filter 155. The light amount of the illumination light L1 is controlled by the diaphragm 156 . The light amount control operation of the illumination light L1 by the diaphragm 156 will be described later.

The CCD 117 that is driven by the CCD drive circuit 118 captures an ordinary image of the region 10 to be observed to obtain an imaging signal, and outputs the imaging signal. The imaging signal is amplified in the CDS/AGC circuit 119 by correlated double sampling and automatic gain control. Further, A/D conversion is performed on the amplified imaging signal in the A/D converter 120, and input to the ordinary image processing unit 374 in the processing unit 372 of the processor unit 370. In the ordinary image processing unit 374, when the ordinary image mode is selected, an ordinary image signal composed of three-color image signals of R, G and B is generated based on the signal output from the scope unit 110. Further, the ordinary image processing unit 374 performs various kinds of processing on the ordinary image signal to generate a Y/C signal (color ordinary image signal) that is composed of a luminance signal Y and chrominance signals C. The Y/C signal is output to the display processing unit 376. In the display processing unit 376, various kinds of signal processing, such as I/P conversion and noise removal processing, are performed on the Y/C signal, and the processed signal is output to the monitor 11.

The monitor 11 displays color ordinary images based on the input color ordinary image signal as a motion image.

Further, the ordinary image processing unit 374 outputs the luminance signal Y for each pixel or an average luminance signal Y' of a plurality of adjacent pixels to the light amount control unit 386. The light amount control unit 386 calculates an average luminance value Ya of pixels in a specified area for each frame based on the input luminance signal Y or average luminance signal Y', and compares the average luminance value Ya with a reference luminance value Yr (standard luminance value) that has been stored in the memory (not illustrated) in advance. Further, the light amount control unit 386 selects a diaphragm control signal based on the result of comparison, and outputs the diaphragm control signal to the control unit 167 of the illumination light unit 150. As the diaphragm control signal, when the average luminance value Ya is greater than the reference luminance value Yr, a signal that reduces the aperture amount of the diaphragm 156 is selected, in other words, a signal that reduces the light passing through the aperture of the diaphragm 156 is selected. When the average luminance value Ya is smaller than the reference luminance value Yr, a signal that increase the aperture amount of the diaphragm 156 is selected, in other words, a signal that increases the light passing through the aperture of the diaphragm 156 is selected. When the average luminance value Ya and the reference luminance value Yr are substantially the same, a signal that maintains the same aperture amount is selected.

The control unit 167 of the illumination light unit 150 controls the aperture amount of the diaphragm 156 based on the diaphragm control signal.

Next, an operation in the fluorescent image mode will be described. Before the fluorescence endoscope apparatus is used in the fluorescent image mode, a washed and sterilized scope unit 110 is attached to the processor unit 370 and the illumination light unit 150 . Further, the connector 127 connected to an end of the signal line 125 and an end of the signal line 126 of the scope unit 110 is connected to the connector 394 of the processor unit 370. When the connector 394 is connected to the connector 127, the connection detection unit 395 provided for the connector 394 outputs a connection signal to the processing unit 372. Further, the optical connector 113 provided at the leading end of the light guide 112 is connected to the optical connector 153 of the illumination light unit 150 . When the optical connector 153 is connected to the connector 113, the connection detection unit 154 provided for the optical connector 153 sends a connection signal to the control unit 167.

Further, the excitation light unit 330 is connected to the illumination light unit 150. The connector 342 provided at the leading end of the signal line 341 of the excitation light unit 330 is connected to the connector 165 of the illumination light unit 150. When the connector 342 is connected to the connector 165, the connection detection unit 166 provided for the connector 165 outputs a connection signal to the control unit 167. Further, the optical connector 336 provided at the leading end of the light guide 333 is connected to the optical connector 161 of the illumination light unit 150. When the optical connector 336 is connected to the optical connector 161, the connection detection unit 162 provided for the optical connector 161 outputs a connection signal to the control unit 167.

The control unit 340 of the excitation light unit 330 communicates with the control unit 167 of the illumination light unit 150. When the control unit 340 receives connection signals from the connection detection unit 166 and the connection detection unit 162, the control unit 340 closes the switch 334 of the excitation light unit 330 to electrically connect the semiconductor laser 331 and the drive circuit 332. Consequently, the semiconductor laser 331 becomes drivable by the drive circuit 332. Further, the function mode of a predetermined key of the input unit 392 is set through the processing unit 372 of the processor unit 370. Further, the function mode of the switch 122 is set through the processing unit 372 and the control unit 121 of the scope unit 110. Since the processing unit 372 controls processing, the operation mode switches between a stop state, an ordinary image mode and a fluorescent image mode when the user presses a predetermined key of the input unit 392 or the switch 122. When the connection signals are not received from both of the connection detection unit 166 and the connection detection unit 162, in other words, when no connection signals are received from the two connection detection units, or no connection signal is received from one of the connection detection units, the switch 334 in the excitation light unit 330 is constantly in an open state. Therefore, when the excitation light unit 330 is not connected to the illumination light unit 150, the semiconductor laser 331 is not driven.

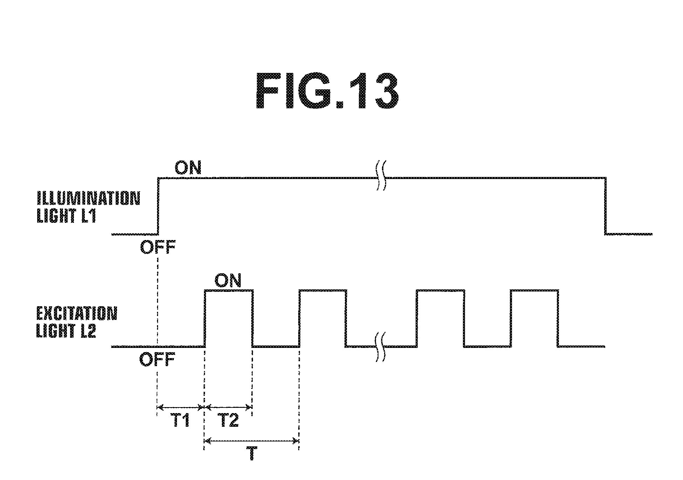

Further, when the fluorescence endoscope apparatus is operating in the ordinary image mode, if the user (doctor) presses the predetermined key of the input unit 392 or the switch 122 once, an operation in the fluorescent image mode starts. In addition to the operation of the illumination light unit 150, the operation of the excitation light unit 330 starts. The semiconductor laser 331 is pulse-driven based on the drive pulse signal output from the drive circuit 332, and excitation light L2 that has a wavelength of 405 nm is output. Specifically, as illustrated in Figure 13, pulsed excitation light that has pulse width of light-output time period T2 is output at cycle T.

The excitation light L2 is condensed by the condensing lens 335, and enters the end surface of the light guide 333. The excitation light L2 propagates through the light guide 333, and enters the light guide 163 through the optical connector 336 and the optical connector 161. The excitation light L2 propagates through the light guide 163 and is output from an end of the light guide 163. The output excitation light L2 is collimated by a collimator lens 164, and enters the dichroic mirror 157. Since the wavelength of the excitation light L2 is 405 nm, the excitation light is reflected at a right angle from the dichroic mirror 157, and condensed onto the end surface of the optical connector 113 by the condensing lens 158 . The condensed light enters the light guide 112.

The excitation light L2 propagates through the light guide 112, and is output from the leading end of the light guide 112. Further, the excitation light L2 is output to the region 10 to be observed through the optical system 111 for illumination to illuminate the region 10 to be observed. At this time, the portion of the region 10 to be observed that is illuminated with the excitation light L2 is illuminated also with the illumination light L1. The light amount of the excitation light L2 is controlled by the drive current from the drive circuit 332 . The operation for controlling the light amount of the excitation light L2 by the drive current will be described later.

The CCD 117 that is driven by the CCD drive circuit 118 captures an ordinary image composed of the reflection light of the illumination light L1, reflected from the region 10 to be observed, in time period T1 illustrated in Figure 13. In the time period T1, only the illumination light L1 is output. Further, in time period T2 illustrated in Figure 13, in which both of the illumination light L1 and the excitation light L2 are output, the CCD 117 captures a combined image composed of the reflection light of the illumination light L1, reflected from the region 10 to be observed, and fluorescence output from the region 10 to be observed by illumination with the excitation light L2. Further, since an excitation light cut filter that cuts light that has a wavelength lower than or equal to 410 nm is provided at the leading end of the CCD 117, most of the reflection light of the excitation light L2 does not enter the CCD 117.

The CCD 117 outputs imaging signals, and the imaging signals are amplified in the CDS/AGC circuit 119 by correlated double sampling and automatic gain control. Further, A/D conversion is performed on the amplified imaging signals in the A/D converter 120, and output as an RGB image signal to the processing unit 372 of the processor unit 370. At this time, the RGB image signal based on the ordinary image is input to the ordinary image processing unit 374, and the RGB image signal based on the combined image is input to the fluorescent image processing unit 382. Further, the ordinary image processing unit 374 generates an ordinary image signal from the input RGB image signal, and outputs the ordinary image signal to the fluorescent image processing unit 382.

Meanwhile, the fluorescent image processing unit 382 generates a combined image signal from the input RGB image signal. Further, the fluorescent image processing unit 382 subtracts the ordinary image signal from the combined image signal to generate a fluorescent image signal. Further, the fluorescent image processing unit 382 performs various kinds of signal processing on the fluorescent image signal, and superimposes the ordinary image signal on the fluorescent image signal to generate a fluorescence superimposed image signal. Further, the fluorescent image processing unit 382 performs various kinds of signal processing on the fluorescence superimposed image signal, and generates a Y/C signal composed of a luminance (Y) signal and chrominance [C (R - Y, B - Y)] signals. The generated Y/C signal is output to the display processing unit 384. The display processing unit 384 performs various kinds of signal processing on the Y/C signal to generate a fluorescence superimposed image signal for display, and outputs the generated fluorescence superimposed image signal for display to the monitor 11.

The monitor 11 displays the fluorescence superimposed image based on the input fluorescent superimposed image signal for display as a motion image. The fluorescence superimposed image is an image in which the fluorescent image and the ordinary image are superimposed one on the other.

Further, the ordinary image processing unit 374 outputs the luminance signal Y for each pixel or an average luminance signal Y' of a plurality of adjacent pixels to the light amount control unit 386. The light amount control unit 386 calculates an average luminance value Ya of pixels in a specified area for each frame, and compares the average luminance value Ya with a reference luminance value Yr (standard luminance value) that has been stored in the memory (not illustrated) in advance. Further, the light amount control unit 386 selects a diaphragm control signal based on the result of comparison, and outputs the diaphragm control signal to the control unit 167 of the illumination light unit 150. As the diaphragm control signal, when the average luminance value Ya is greater than the reference luminance value Yr, a signal that reduces the aperture amount of the diaphragm 156 is selected, in other words, a signal that reduces the light passing through the aperture of the diaphragm 156 is selected. When the average luminance value Ya is smaller than the reference luminance value Yr, a signal that increase the aperture amount of the diaphragm 156 is selected, in other words, a signal that increases the light passing through the aperture of the diaphragm 156 is selected. When the average luminance value Ya and the reference luminance value Yr are substantially the same, a signal that maintains the same aperture amount is selected. Further, a drive current control signal corresponding to the diaphragm control signal is output so that the ratio of the light amount of the illumination light L1 to the light amount of the excitation light L2 becomes a predetermined ratio. The ratio of the light amount of the illumination light L1 to the light amount of the excitation light L2 may be set by an input operation at the input unit 392 in advance. The light amount control unit 386 determines the drive current amount of the semiconductor laser 331 based on the set ratio and the aperture amount of the illumination light L1, and outputs the drive current control signal.

The control unit 167 of the illumination light unit 150 controls the aperture amount of the diaphragm 156 based on the diaphragm control signal. Further, the control unit 340 of the excitation light unit 330 controls, based on the drive current control signal, the value of the electric current supplied from the drive circuit 332 to the semiconductor laser 331.

In the above description, the fluorescent image signal and the ordinary image signal were superimposed one on the other to generate the fluorescence superimposed image signal. Further, the fluorescence superimposed image for display was displayed based on the fluorescence superimposed image signal. However, it is not necessary that the processing is performed in such a manner. For example, a fluorescence yield may be obtained by dividing the fluorescent image signal by the value of the luminance signal Y of the ordinary image signal. Further, the colors of red, yellow and green may be sequentially assigned to the fluorescence yield based on the size (value) of the fluorescence yield to produce a fluorescent image for display. Further, the produced fluorescent image for display may be displayed on the monitor 11.

Further, the characteristic that the intensity of fluorescence output from normal tissue and the intensity of fluorescence output from lesion tissue differ from each may be utilized, and a judgment unit that judges whether the region 10 to be observed is a lesion tissue based on the fluorescence yield that has been obtained as described above may be provided. When the judgment unit has judged that the region 10 to be observed is a normal tissue, an ordinary image may be displayed. When the judgment unit has judged that the region 10 to be observed is a lesion tissue, a new fluorescent image may be produced by changing the ordinary color image of the pixel based on the fluorescence yield, and displayed.

Further, with respect to the ordinary image, a new ordinary image may be produced by applying a predetermined gain and/or offset, and the produced image may be displayed. In this case, it is desirable that the gain and/or offset for the ordinary image differs from the gain and/or offset for the fluorescent image. For example, the gains and the offsets may satisfy the following relations: gain GAINf for the fluorescent image > gain GAINn for the ordinary image; and offset OFFSETf for the fluorescent image > offset OFFSETn for the ordinary image.

Further, the fluorescent image signal may be added to the combined image signal to enhance the fluorescence, and the fluorescent image for display may be produced.

Further, in the fluorescence endoscope apparatus of the above embodiments, the ordinary image signal is generated based on the ordinary image obtained in the time period when only the illumination light L1 is output, and the fluorescent image signal is generated based on the combined image obtained in the time period when both of the illumination light L1 and the excitation light L2 are output. Since the time period in which the ordinary image is obtained and the time period in which the combined image is obtained differ from each other, the imaging position of the region 10 to be observed corresponding to the ordinary image and the imaging position of the region 10 to be observed corresponding to the combined image may differ from each other in some cases. In such cases, the shift (difference) in the imaging positions may be detected based on the ordinary image signal based on the ordinary image and the combined image signal based on the combined image. Further, positioning may be performed based on the shift amount in position. For example, the positions may be adjusted by performing shift correction processing on one of the image signals.

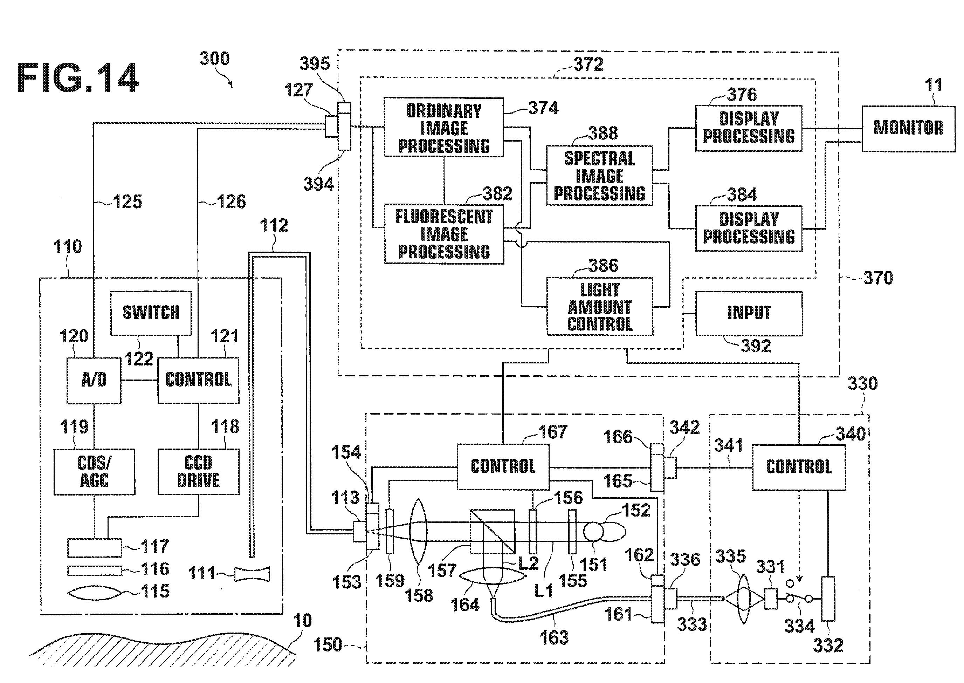

Further, as illustrated in Figure 14, a spectral image processing unit 388 may be provided in the fluorescence endoscope apparatus of the above embodiments. The spectral image processing unit 388 may perform spectral image processing on at least one of the ordinary image signal composed of RGB image signals, obtained in the ordinary image processing unit 374, the combined image signal composed of RGB image signals, obtained in the fluorescent image processing unit 382, and the fluorescent image signal obtained in the fluorescent image processing unit 382. Further, whether the spectral image processing is performed may be selected by an input operation at the input unit 392. The action in the case of performing spectral image processing on the ordinary image signal, the fluorescent image signal or the combined image signal is similar to the action of the fluorescence endoscope apparatus of the second embodiment.

Further, the spectral image processing unit 388 may obtain a fluorescence yield, and produce a fluorescent image based on the fluorescence yield.

Specifically, the spectral image processing unit 388 uses, as reference light intensity, the value of the luminance signal Y obtained by the spectral image processing on the ordinary image signal. Further, the spectral image processing unit 388 divides the luminance signal Y' obtained by spectral image processing on the spectral image by the value of the luminance signal to obtain an estimated fluorescence yield. For example, a fluorescence image for display is produced by sequentially assigning red, yellow or green to the estimated fluorescence yield, as illustrated in Figure 5. In this case, the lesion tissue that has a smaller estimated fluorescence yield is display in red, and a normal tissue that has a larger estimated fluorescence yield is displayed in green. When the estimated fluorescence yield is lower than or equal to a predetermined lower limit value, only red is assigned. When the estimated fluorescence yield is greater than or equal to a predetermined upper limit value, green is assigned. Alternatively, as illustrated in Figure 6, red, yellow, green, cyan or blue may be assigned to the estimated fluorescence yield. Further, when the estimated fluorescence yield is less than or equal to a predetermined lower limit value or higher than or equal to a predetermined upper limit value, an achromatic color may be assigned.

In the present embodiment, the intensity of light obtained from estimated spectral data in a long wavelength band, for example at 620 nm or the like, may be used as the reference light intensity in a manner similar to the second embodiment. In the long wavelength band, a difference between the intensity of fluorescence output from the normal tissue and the intensity of fluorescence output from the lesion tissue is small.

For example, the spectral image processing unit 388 generates fluorescence superimposed image for display so that the user who observes the image can easily identify or confirm the position of the lesion tissue, which has a small estimated fluorescence yield. The fluorescence superimposed image for display is produced by assigning data that has been obtained by applying an arbitrary gain or offset to the estimated fluorescence yield to one or two of R, G and B to produce a fluorescent image, and by superimposing the fluorescent image on a black-and-white ordinary image that reflects the luminance signal Y of the ordinary image. The obtained fluorescence superimposed image for display is output to the display processing unit 384. The display processing unit 384 may produce a display image in which the color ordinary image and the fluorescence superimposed image for display that have been output from the spectral image processing unit 388 are displayed next to each other, and output the display image to the monitor 11 to display the image.

Further, the embodiments of the fluorescent image obtainment apparatus of the present invention are not limited to the aforementioned embodiments. The fluorescent image obtainment apparatus of the present invention may be embodied in any manner as long as illumination light and excitation light are output and a fluorescent image is obtained. For example, the fluorescent image obtainment apparatus of the present invention may be an endoscope apparatus including a light source unit, such as an LED, which is provided at the leading end of the scope unit of the endoscope, a colposcope, a capsule endoscope apparatus or the like. Further, the fluorescent image obtainment apparatus of the present invention may be a microscope having a fluorescent image obtainment function or the like.

In the above description, a primary-color-type three-color filter is used as the mosaic filter of the CCD 117. However, the filter is not limited to the primary-color-type three-color filter. A four-color type mosaic filter, a complementary-color-type mosaic filter or the like may be used instead of the primary-color-type three-color filter. In this case, the signal output from the CCD 117 may be converted into a primary-color-type signal by signal processing. Alternatively, estimation matrix data corresponding to the spectral characteristics of these mosaic filters may be stored in the memory in advance.

In the embodiments of the present invention, autofluorescence that is output from the living tissue of a patient was obtained. However, it is not necessary that the autofluorescence is obtained. For example, fluorescence output from a region to be observed to which a fluorescent dye, such as indocyanine green, has been injected may be obtained. Further, as the excitation light, light that has a wavelength of 405 nm was used. However, it is not necessary that the excitation light has such a wavelength. For example, when indocyanine green is used as the fluorescent dye, excitation light in the wavelength band of 700 nm to 800 nm may be used. In that case, the wavelength band of fluorescence output from the indocyanine green is longer than or equal to 800 nm. Further, the type of the excitation light source is not limited to the laser. An LED may be used as the excitation light source.

Preferred embodiments of the invention are outlined in the following paragraphs:

1. A fluorescent image obtainment method comprising the steps of:

capturing an image composed of light reflected from a region to be observed, the region having been illuminated with illumination light, and an image composed of fluorescence output from the region to be observed, the region having been illuminated with excitation light at the same time as illumination with the illumination light;

producing an ordinary image based on the captured image composed of the reflection light; and

producing a fluorescent image based on the captured image composed of fluorescence, wherein the light amount of the illumination light is controlled so that a representative luminance value of the ordinary image becomes a predetermined luminance value, and wherein the light amount of the excitation light is controlled so that the ratio of the light amount of the excitation light to the light amount of the illumination light becomes a predetermined ratio.

2. A fluorescent image obtainment method comprising the steps of:

driving an illumination light source means so as to continuously output illumination light;

pulse-driving an excitation light source means, based on a pulse signal, so as to output pulsed excitation light;

capturing an image composed of light reflected from a region to be observed by illumination with the illumination light, and an image including fluorescence output from the region to be observed by illumination with excitation light;

producing an ordinary image based on the captured image composed of the reflection light; and

producing a fluorescent image based on the captured image including fluorescence, wherein the light amount of the illumination light is controlled so that a representative luminance value of the ordinary image becomes a predetermined luminance value, and wherein the light amount of the excitation light is controlled by controlling the pulse signal so that the ratio of the light amount of the excitation light to the light amount of the illumination light becomes a predetermined ratio.

3. A fluorescent image obtainment method comprising the steps of:

driving an illumination light source means so as to continuously output illumination light;

pulse-driving an excitation light source means so as to output pulsed excitation light;

continuously illuminating a region to be observed with the illumination light;

illuminating a region that is substantially the same as the region illuminated with the illumination light with the pulsed excitation light;

capturing a first image composed of light reflected from the region to be observed in a time period when the region to be observed is illuminated only with the illumination light in the process of continuously illuminating the region to be observed with the illumination light and illuminating substantially the same region with the pulsed excitation light;

capturing a second image composed of light reflected from the region to be observed and fluorescence output from the region to be observed in a time period when the region to be observed is illuminated with both of the illumination light and the excitation light in the process of continuously illuminating the region to be observed with the illumination light and illuminating substantially the same region with the pulsed excitation light;

producing an ordinary image based on the first image; and

producing a fluorescent image based on the first image and the second image.

4. A fluorescent image obtainment method comprising the steps of:

capturing an image composed of light reflected from a region to be observed, the region having been illuminated with illumination light, and fluorescence output from the region to be observed, the region having been illuminated with excitation light at the same time as illumination with the illumination light;

calculating estimated spectral data of a specific fluorescence wavelength band for each pixel of image signals of the captured image, the specific fluorescence wavelength band including at least an approximate center wavelength band of the fluorescence, and the estimated spectral data being calculated based an image signal value of each of the pixels and an estimation matrix for calculating the estimated spectral data, and the estimation matrix having been stored in advance;

obtaining, based on the estimated spectral data of the specific fluorescence wavelength band, information that reflects the radiation intensity of the fluorescence output from the region to be observed; and

producing a fluorescent image based on the information that reflects the radiation intensity of the fluorescence.

5. A fluorescent image obtainment apparatus comprising:

a light illumination means (130, 150) that illuminates a region to be observed with illumination light and excitation light at the same time;

an imaging means (110) that captures an image composed of light reflected from the region to be observed, the region having been illuminated with the illumination light, and an image composed of fluorescence output from the region to be observed, the region having been illuminated with the excitation light;

an image processing means (170) that produces an ordinary image based on the image composed of the reflection light, the image having been captured by the imaging means, and a fluorescent image based on the image composed of fluorescence, the image having been captured by the imaging means; and

a light amount control means (186) that controls the light amount of the illumination light so that a representative luminance value of the ordinary image becomes a predetermined luminance value, and that controls the light amount of the excitation light so that the ratio of the light amount of the excitation light to the light amount of the illumination light becomes a predetermined ratio.

6. A fluorescent image obtainment apparatus, as defined in para 5, the apparatus further comprising:

a storage unit (190) that stores an estimation matrix for pseudo-spectral image processing; and

an estimated spectral data calculation means (180) that uses an image signal output from the imaging means and the estimation matrix, and calculates, for each pixel of the image signal, estimated spectral data, wherein the image processing means (170) obtains, based on the estimated spectral data for each of the pixels, estimated spectral data of a specific fluorescence wavelength band that includes at least a center wavelength band of the fluorescence, and produces the fluorescent image based on the estimated spectral data of the specific fluorescence wavelength band.

7. A fluorescent image obtainment apparatus, as defined in para 6, wherein the image processing means (170):

calculates, based on the estimated spectral data of the specific fluorescence wavelength band, pseudo-fluorescence intensity, which is the intensity of light in the specific fluorescence wavelength band;

obtains estimated spectral data of a reference wavelength band that does not include the specific fluorescence wavelength band;

calculates, based on the estimated spectral data of the reference wavelength band, reference light intensity, which is the intensity of light in the reference wavelength band;

calculates a pseudo-fluorescence yield by dividing the pseudo-fluorescence intensity by the reference light intensity; and

produces the fluorescent image based on the pseudo-fluorescence yield.

8. A fluorescent image obtainment apparatus comprising:

an illumination light source means (150) that continuously outputs illumination light;

an excitation light source means (230) that is pulse-driven based on a pulse signal to output pulsed excitation light;

an illumination means (110) that illuminates substantially the same position of a region to be observed with the illumination light and the excitation light;

an imaging means (110) that captures an image composed of light reflected from the region to be observed by illumination with the illumination light, and an image including fluorescence output from the region to be observed by illumination with the excitation light;

an image processing means (270) that produces an ordinary image based on the image composed of the reflection light, the image having been captured by the imaging means (110), and that produces a fluorescent image based on the image including fluorescence, the image having been captured by the imaging means (110); and

a light amount control means (286) that controls the light amount of the illumination light so that a representative luminance value of the ordinary image becomes a predetermined luminance value, and that controls the light amount of the excitation light by controlling the pulse signal so that the ratio of the light amount of the excitation light to the light amount of the illumination light becomes a predetermined ratio.

9. A fluorescent image obtainment apparatus, as defined in para 8, wherein the excitation light source means (230) outputs, at a predetermined cycle, one or a plurality of beams of pulsed excitation light in a predetermined time period in which the excitation light can be output, and wherein the light amount control means (286) controls the light amount of the excitation light by changing the number of beams of the pulsed excitation light in the predetermined time period in which the excitation light can be output and/or the light output time period of the pulsed excitation light in the predetermined time period in which the excitation light can be output.

10. A fluorescent image obtainment apparatus, as defined in any one of paras 5 to 9, further comprising:

a setting means (292) that sets the ratio of the light amount of the excitation light to the light amount of the illumination light by an input operation.

11. A fluorescent image obtainment apparatus comprising:

an illumination light source means (150) that continuously outputs illumination light;

an excitation light source means (330) that is pulse-driven based on a pulse signal to output pulsed excitation light;

an illumination means (110) that illuminates substantially the same position of a region to be observed with the illumination light and the excitation light:

an imaging means (110) that captures a first image composed of light reflected from the region to be observed in a time period when the region to be observed is illuminated only with the illumination light and that captures a second image composed of light reflected from the region to be observed and fluorescence output from the region to be observed in a time period when the region to be observed is illuminated with both of the illumination light and the excitation light; and

an image processing means (370) that produces an ordinary image based on the first image and that produces, based the ordinary image and a combined image that is based on the second image, a fluorescent image.

12. A fluorescent image obtainment apparatus, as defined in para 11, wherein the image processing means (370) produces an image for display by performing an operation by using at least two of the ordinary image, the fluorescent image and the combined image.

13. A fluorescent image obtainment apparatus, as defined in para 12, wherein the image processing means (370) produces, based on the luminance ratio between the ordinary image and the fluorescent image, the image for display.

14. A fluorescent image obtainment apparatus, as defined in any one of paras 11 to 13, wherein the image processing means (370) performs spectral image processing on at least one of the ordinary image, the fluorescent image and the combined image.

15. A fluorescent image obtainment apparatus, as defined in para 14, further comprising:

a selection means (392) that can select whether the spectral image processing is performed on at least one of the ordinary image, the fluorescent image and the combined image.

16. A fluorescent image obtainment apparatus comprising:

a light illumination means (150, 130) that illuminates a region to be observed with illumination light and excitation light at the same time;

an imaging means (110) that captures an image composed of light reflected from the region to be observed by illumination with the illumination light, and fluorescence output from the region to be observed by illumination with the excitation light;

a storage unit (190) that stores an estimation matrix for calculating estimated spectral data; and

an image processing means (170) that calculates estimated spectral data of a specific fluorescence wavelength band for each pixel of an image signal that has been output from the imaging means, the specific fluorescence wavelength band including at least an approximate center wavelength band of the fluorescence, and the estimated spectral data being calculated based an image signal value of each of the pixels and the estimation matrix, and obtains, based on the estimated spectral data of the specific fluorescence wavelength band, information that reflects the radiation intensity of the fluorescence output from the region to be observed, and produces a fluorescent image based on the information that reflects the radiation intensity of the fluorescence.

17. A fluorescent image obtainment apparatus, as defined in para 16, wherein the light illumination means (150, 130) illuminates the region to be observed with reference light that has a wavelength different from that of the excitation light at the same time as illuminating the region to be observed with the excitation light, and wherein the imaging means (110) captures an image including reflection light of the reference light, the reflection light having been reflected from the region to be observed, and wherein the image processing means (170) calculates reference light intensity that is the intensity of reflection light of the reference light, the reflection light being included in the image captured by the imaging means, and calculates, based on the estimated spectral data of the specific fluorescence wavelength band, pseudo-fluorescence intensity, which is the intensity of light in the specific fluorescence wavelength band, and calculates a pseudo-fluorescence yield, as radiation intensity information about the fluorescence, by dividing the pseudo-fluorescence intensity by the reference light intensity.

18. A fluorescent image obtainment apparatus, as defined in para 16 or 17, wherein the image processing means (170) obtains, for each pixel of the image signal output from the imaging means, estimated spectral data of a semi-ordinary wavelength band that does not include the specific fluorescence wavelength band by using the image signal value of each of the pixels and the estimation matrix, and produces a pseudo-ordinary image based on the estimated spectral data of the semi-ordinary wavelength band.

19. A fluorescent image obtainment apparatus, as defined in para 18, the apparatus further comprising:

a display processing means (184) that produces a fluorescence superimposed image in which the fluorescent image is superimposed on the pseudo-ordinary image.

20. A fluorescent image obtainment apparatus, as defined in any one of paras 16 to 19, the apparatus further comprising:

an input means (192) that sets the specific fluorescence wavelength band by an input operation.

21. A fluorescence endoscope apparatus comprising:

an illumination light unit (150) that includes a light source for illumination light that outputs the illumination light;

an excitation light unit (130) that is detachably connected to the illumination light unit (150) and that includes a light source (131) for excitation light that outputs the excitation light;

a first light guide means (110) that guides the illumination light or the excitation light to illuminate a region to be observed with the illumination light or the excitation light;

an imaging means (110) that captures an image composed of light reflected from the region to be observed by illumination with the illumination light or an image composed of fluorescence output from the region to be observed by illumination with the excitation light;

an image processing means (170) that produces an ordinary image based on the image composed of the reflection light, the image having been captured by the imaging means (110), and that produces a fluorescent image based on the image composed of the fluorescence, the image having been captured by the imaging means (110);

a detection means (162, 166) that detects whether the excitation light unit (130) is connected to the illumination light unit (150); and

a light-output prevention means (134) that prevents the excitation light unit (130) from outputting the excitation light when the detection means (162, 166) has not detected that the excitation light unit (130) is connected to the illumination light unit (150).

22. A fluorescence endoscope apparatus, as defined in para 21, wherein the illumination light unit (150) includes a second light guide means (161) that guides the excitation light, and wherein the excitation light unit (130) includes a third light guide means (133, 136) that extends to the outside of the excitation light unit (130) and guides the excitation light, and wherein the detection means (162) detects whether the second light guide means (161) and the third light guide means (133, 136) are optically connected to each other.

23. A fluorescence endoscope apparatus, as defined in para 21 or 22, wherein the excitation light unit (130) includes a drive unit (132) for excitation light that drives the light source (131) for excitation light, and wherein when the light-output prevention means (134) prevents the excitation light unit (130) from outputting the excitation light, the light-output prevention means (134) prevents a drive unit (132) for excitation light and the light source (131) for excitation light from being electrically connected to each other.

24. A fluorescence endoscope apparatus, as defined in any one of paras 21 to 23, wherein the illumination light unit (150) includes a drive unit (152) for illumination light that drives the light source (151) for illumination light and an illumination light control unit (167) that controls the drive unit (152) for illumination light, and wherein the excitation light unit (130) includes a drive unit (132) for excitation light that drives the light source (131) for excitation light and an excitation light control unit (140) that controls the drive unit (132) for excitation light, and wherein the detection means (166) further detects whether the illumination light control unit (167) and the excitation light control unit (140) are electrically connected to each other.

25. A fluorescence endoscope apparatus, as defined in any one of paras 21 to 24, wherein an operation is performed in an ordinary image mode or in a fluorescent image mode, and wherein in the ordinary image mode, the illumination light is output and the ordinary image is produced, and wherein in the fluorescent image mode, the excitation light is output and the fluorescent image is produced, and wherein when the detection means (162, 166)has not detected that the excitation light unit (130) is connected to the illumination light unit (150), the operation in the fluorescent image mode is not performed.

26. An excitation light unit (130) detachably connected to an illumination light unit (150) of an endoscope apparatus (100), wherein the endoscope apparatus (100) includes:

the illumination light unit (150) that has a light source for illumination light that outputs illumination light;

a first light guide means (110) that guides the illumination light or excitation light to illuminate a region to be observed with the illumination light or the excitation light;

an imaging means (110) that captures an image of the region to be observed that has been illuminated with the illumination light or a fluorescence image of the region to be observed that has been illuminated with the excitation light; and

an image processing means (170) that produces an ordinary image based on the image of the region to be observed, the image having been captured by the imaging means (110), and that produces a fluorescent image based on the fluorescence image of the region to be observed, the fluorescence image having been captured by the imaging means,

the excitation light unit (130) comprising:

a light source (131) for excitation light that outputs the excitation light;

a detection means (162, 166) that detects whether the excitation light unit (130) is connected to the illumination light unit (150); and

a light-output prevention means (134) that prevents the excitation light unit (150) from outputting the excitation light to the outside of the excitation light unit (150) when the detection means (162, 166) has detected that the excitation light unit (130) is not connected to the illumination light unit (150).

**Claims**

1. A fluorescent image obtainment method comprising the steps of:

capturing an image composed of light reflected from a region to be observed, the region having been illuminated with illumination light, and an image including fluorescence output from the region to be observed, the region having been illuminated with excitation light;
producing an ordinary image based on the captured image composed of the reflection light; and
producing a fluorescent image based on the captured image composed of fluorescence, wherein the light amount of the illumination light is controlled so that a representative luminance value of the ordinary image becomes a predetermined luminance value, and wherein the light amount of the excitation light is controlled so that the ratio of the light amount of the excitation light to the light amount of the illumination light becomes a predetermined ratio.

2. A fluorescent image obtainment method as defined by claim 1, wherein the region has been illuminated with excitation light at the same time as illuminated with illumination light.

3. A fluorescent image obtainment method as defined in claim 1 or 2, further comprising the steps of:

driving an illumination light source means so as to continuously output the illumination light;
pulse-driving an excitation light source means, based on a pulse signal, so as to output pulsed excitation light; and
wherein the light amount of the excitation light is controlled by controlling the pulse signal so that the ratio of the light amount of the excitation light to the light amount of the illumination light becomes a predetermined ratio.

4. A fluorescent image obtainment apparatus comprising:

a light illumination means (130, 150) that illuminates a region to be observed with illumination light and excitation light;
an imaging means (110) that captures an image composed of light reflected from the region to be observed, the region having been illuminated with the illumination light, and an image including fluorescence output from the region to be observed, the region having been illuminated with the excitation light;
an image processing means (170) that produces an ordinary image based on the image composed of the reflection light, the image having been captured by the imaging means, and a fluorescent image based on the image including fluorescence, the image having been captured by the imaging means; and
a light amount control means (186) that controls the light amount of the illumination light so that a representative luminance value of the ordinary image becomes a predetermined luminance value, and that controls the light amount of the excitation light so that the ratio of the light amount of the excitation light to the light amount of the illumination light becomes a predetermined ratio.

5. A fluorescent image obtainment apparatus as defined by claim 4, wherein said light illumination means (130, 150) is adapted to illuminate the region with illumination light and excitation light at the same time.

6. A fluorescent image obtainment apparatus, as defined in claim 4 or 5, the apparatus further comprising:

a storage unit (190) that stores an estimation matrix for pseudo-spectral image processing; and
an estimated spectral data calculation means (180) that uses an image signal output from the imaging means and the estimation matrix, and calculates, for each pixel of the image signal, estimated spectral data, wherein the image processing means (170) obtains, based on the estimated spectral data for each of the pixels, estimated spectral data of a specific fluorescence wavelength band that includes at least a center wavelength band of the fluorescence, and produces the fluorescent image based on the estimated spectral data of the specific fluorescence wavelength band.

7. A fluorescent image obtainment apparatus, as defined in claim 6, wherein the image processing means (170):

calculates, based on the estimated spectral data of the specific fluorescence wavelength band, pseudo-fluorescence intensity, which is the intensity of light in the specific fluorescence wavelength band;
obtains estimated spectral data of a reference wavelength band that does not include the specific fluorescence wavelength band;
calculates, based on the estimated spectral data of the reference wavelength band, reference light intensity, which is the intensity of light in the reference wavelength band;
calculates a pseudo-fluorescence yield by dividing the pseudo-fluorescence intensity by the reference light intensity; and
produces the fluorescent image based on the pseudo-fluorescence yield.

8. A fluorescent image obtainment apparatus as defined by claim 4, wherein said light illumination means comprises:

an illumination light source means (150) that continuously outputs illumination light;
an excitation light source means (230) that is pulse-driven based on a pulse signal to output pulsed excitation light;
an illumination means (110) that illuminates substantially the same position of a region to be observed with the illumination light and the excitation light; and wherein
said light amount control means (286) is adapted to control the light amount of the illumination light so that a representative luminance value of the ordinary image becomes a predetermined luminance value, to control the light amount of the excitation light by controlling the pulse signal so that the ratio of the light amount of the excitation light to the light amount of the illumination light becomes a predetermined ratio.

9. A fluorescent image obtainment apparatus, as defined in claim 8, wherein the excitation light source means (230) outputs, at a predetermined cycle, one or a plurality of beams of pulsed excitation light in a predetermined time period in which the excitation light can be output, and wherein the light amount control means (286) controls the light amount of the excitation light by changing the number of beams of the pulsed excitation light in the predetermined time period in which the excitation light can be output and/or the light output time period of the pulsed excitation light in the predetermined time period in which the excitation light can be output.

10. A fluorescent image obtainment apparatus, as defined in any one of claims 4 to 9, further comprising:

a setting means (292) that sets the ratio of the light amount of the excitation light to the light amount of the illumination light by an input operation.

# FIG.1

EP 2 404 543 A1

# FIG.2A

INTENSITY

WAVELENGTH (nm)

400    500    600    700

# FIG.2B

INTENSITY

WAVELENGTH (nm)

400    500    600    700

FIG.3

FIG.4

# FIG.5

PSEUDO-FLUORESCENCE YIELD

| | R | G | |

LOW     LOWER        JUDGMENT        UPPER    HIGH
         LIMIT          VALUE          LIMIT
         VALUE                         VALUE

LUMINANCE

R                     G

# FIG.6

PSEUDO-FLUORESCENCE YIELD

| | R | G | B | |

LOW     LOWER    JUDGMENT        JUDGMENT    UPPER    HIGH
         LIMIT     VALUE 1         VALUE 2     LIMIT
         VALUE                                    VALUE

LUMINANCE

R          G             B

FIG.7

EP 2 404 543 A1

**100**

**195**

**127**

**172**

ORDINARY IMAGE PROCESSING — 174

DISPLAY PROCESSING — 176

MONITOR — 11

194

ESTIMATED SPECTRAL DATA CALCULATION — 180

IMAGE PROCESSING — 182

DISPLAY PROCESSING — 184

125 126

112

110

170

SWITCH

MEMORY

LIGHT AMOUNT CONTROL — 186

INPUT

120 122 121

190

192

A/D CONTROL

130

119 118

142

141

140

CDS/ AGC

CCD DRIVE

154

CONTROL — 167

166

CONTROL

113

165

117

116

111

115

153 159 156a 157 155 151 162 136 135 131 134 132

158 164 161 133

163

10

150

152

111

FIG.8

# FIG.9

# FIG.10

FIG.11

EP 2 404 543 A1

FIG.12

# FIG.13

ILLUMINATION LIGHT L1

EXCITATION LIGHT L2

ON

OFF

ON

OFF

T1

T2

T

FIG.14

FIG.15

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 749 830 A (KANEKO MAMORU [JP] ET AL) 12 May 1998 (1998-05-12) | 1,3,4, 6-10 | INV. A61B1/06 |
| Y | * column 7, line 21 - line 57 * * column 15, line 26 - line 43 * * column 20, line 1 - line 9 * * column 23, line 7 - column 24, line 67 * * figure 2 * ----- | 2,5 | A61B5/00 A61B1/04 A61B1/07 |
| Y,D | EP 1 813 185 A (FUJINON CORP [JP]) 1 August 2007 (2007-08-01) * paragraph [0016] - paragraph [0051] * * figure 1 * ----- | 2,5 | |

TECHNICAL FIELDS
SEARCHED      (IPC)

A61B

The present search report has been drawn up for all claims

| Place of search | Date of completion of the search | Examiner |
|---|---|---|
| Munich | 2 December 2011 | Montes, Pau |

**ANNEX TO THE EUROPEAN SEARCH REPORT
ON EUROPEAN PATENT APPLICATION NO.**

EP 11 17 9380

This annex lists the patent family members relating to the patent documents cited in the above-mentioned European search report.
The members are as contained in the European Patent Office EDP file on
The European Patent Office is in no way liable for these particulars which are merely given for the purpose of information.

02-12-2011

| Patent document cited in search report | | Publication date | Patent family member(s) | | Publication date |
|---|---|---|---|---|---|
| US 5749830 | A | 12-05-1998 | NONE | | |
| EP 1813185 | A | 01-08-2007 | EP | 1813185 A1 | 01-08-2007 |
| | | | JP | 4773217 B2 | 14-09-2011 |
| | | | JP | 2007202621 A | 16-08-2007 |
| | | | US | 2007183162 A1 | 09-08-2007 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6465968 B
- US 20030216626 A
- US 20040162492 A
- JP 3718024 B
- JP 2002143079 A
- US 20060256191 A
- JP 2003093336 A
- US 20070183162 A